# EUROPEAN PATENT SPECIFICATION

(11) **EP 4 587 824 B1**
(45) Date of publication and mention of the grant of the patent: **28.10.2026**
(21) Application number: 23765545.1
(22) Date of filing: 12.09.2023
(51) Int. Cl.: G01N 30/88, G01N 30/10, G01N 30/46, G01N 33/15, C07K 16/00, C07K 16/32, G01N 30/06

(54) **A MULTIDIMENSIONAL LC SYSTEM FOR ANALYSING ANTIBODIES**
MEHRDIMENSIONALES LC-SYSTEM ZUR ANALYSE VON ANTIKÖRPERN
SYSTÈME LC MULTIDIMENSIONNEL POUR ANALYSER DES ANTICORPS

(30) Priority: 13.09.2022 EP 22195462
(43) Date of publication of application: 23.07.2025
(73) Proprietor: F. Hoffmann-La Roche AG, 4070 Basel (CH)
(72) Inventor: BATHKE, Anja, 4070 Basel (CH); HOELTERHOFF, Sina Nadine, 4070 Basel (CH); OEZIPEK, Saban, 4070 Basel (CH)
(74) Representative: Mewburn Ellis LLP
(86) International application number: PCT/EP2023/075057
(87) International publication number: WO 2024/056686

(56) References cited:
- VERSCHEURE LIESA ET AL: "3D-LC-MS with 2 D Multimethod Option for Fully Automated Assessment of Multiple Attributes of Monoclonal Antibodies Directly from Cell Culture Supernatants", ANALYTICAL CHEMISTRY, vol. 94, no. 17, 3 May 2022 (2022-05-03), US, pages 6502 - 6511, XP093019234, ISSN: 0003-2700, DOI: 10.1021/acs.analchem.1c05461
- CAMPERI JULIEN ET AL: "Targeted Bottom-up Characterization of Recombinant Monoclonal Antibodies by Multidimensional LC/MS", ANAL. CHEM., vol. 92, 9 September 2020 (2020-09-09), pages 13420 - 13426, XP093019459
- OEZIPEK, S.HOELTERHOFF, SBREUER, S.BELL, C.BATHKE, A.: "mD-UPLC-MS/MS: Next Generation of mAb Characterization by Multidimensional Ultraperformance Liquid Chromatography-Mass Spectrometry and Parallel On-Column LysC and Trypsin Digestion", ANALYTICAL CHEMISTRY, vol. 94, 12 May 2022 (2022-05-12), pages 8136 - 8145, XP002808563

## Description

### Background

Therapeutic monoclonal antibodies (mAbs) have become increasingly important for the treatment of critical diseases. To ensure patient safety it is crucial that quality control confirms the reliability and consistency of biopharmaceutical products such as mAbs across the entire product life cycle.

For example, protein stability has to be maintained from production until application, to assure a safe and efficacious treatment of patients. To provide sufficient quality of biopharmaceutical products, the U.S. Food and Drug Administration (FDA) recommends characterization and monitoring of critical quality attributes (CQAs).

To comply with the requirements three different measurements of mAbs have become standard for quality control of biopharmaceuticals. The three measurements are native mAb analysis, reduced mAb analysis and peptide mapping analysis of the mAb.

The combination of these three measurements allows the accurate identification of fragments and post-translational modifications (PTMs).

Measurements of mAbs at the native and reduced level provides protein level information on the mAb. The most common changes at the protein level include the occurrence of high molecular weight aggregates (HMWs) such as mAb dimers, by-products that may result from formulation (e.g. derived from incorrect light or heavy chain association), and low molecular weight fragments (LMWs). In the case of bispecific antibodies in particular, such changes and their measurement often identify critical product-related contamination.

Native level measurement provides information about structural details of the complex at the protein level, such as conformation and glycosylation and size variants. It is important to monitor these features for example size variants can cause immunogenic response or may have differences in pharmacokinetics or potency compared to the desired product. Additionally, data acquisition and interpretation at the native level may be faster and less complex compared to peptide mapping and so provide an initial picture of quality more quickly.

Reduced level measurements provide additional information on the light and heavy chain (LC and HC) and possible degradation fragments of both chains. At this level the internal disulfide bridges are reduced. It may be easier to carry out these measurements compared to the, larger, native level measurements. Reduced level measurements also enable the detection and identification of some post translational modification such as glycosylation, oxidation, cyclization and deamidation and provide their location on the chains and degradation fragments.

Measurement of mAbs at the peptide level provide information on specific chemical amino acid modifications and their exact location. The most common degradations are oxidation of methionine and tryptophan, deamidation of asparagine, isomerization of aspartate, and glycation of lysine residues. Monitoring of these specific modifications is important, because they show altered stability as well as an impact on biological function. This is especially the case for deamidation of asparagine residues located in the complementarity-determining regions (CDRs) and methionine oxidation as oxidization of monoclonal antibodies (mAbs) is known to influence in vivo product stability, receptor binding, and structure and exhibit accelerated plasma clearance.

In order to provide measurements at multiple levels (native, reduced and peptide), separate measurement protocols are currently used. These separate steps are time consuming and labour intensive. In the case of native and reduced measurements, typically the preparation of the sample and the measurements are manually performed. For peptide mapping, some automated systems for preparing and measuring the sample have been developed.

At the native level, the state of the art processes for measurements are typically mass spectrometer (MS) analysis of the native mAbs in an appropriate buffer.

At the reduced level, measurements can be carried out manually by separate reduction reactions followed by mass spectrometer analysis of the cleaned reduced sample. It is also known to perform online reduction and MS analysis in a liquid chromatography system coupled to a mass spectrometer (see Bathke et al. (Bathke, A., Klemm, D., Gstottner, C., Bell, C., & Kopf, R. (2018), "Rapid Online Reduction and Characterization of Protein Modifications Using Fully Automated Two-Dimensional High Performance Liquid Chromatography-Mass Spectrometry", LC-GC North America, 36(3), 18-29.).

At the peptide measurement level, automated sample preparation and peptide mapping analysis by liquid chromatography (LC) based methods have been developed. In these cases the sample is injected directly into a multidimensional LC-system (mD-LC) and the sample is processed and analyzed in an automated manner.

These LC based methods can combine chromatographic steps (e.g. ion exchange chromatography (IEC), size exclusion chromatography (SEC), Protein-A), reductions steps or digestion steps as needed prior to peptide mapping in an automated manner. The different steps are referred to as "dimensions".

Known multidimensional LC systems for peptide mapping include the system of Oezipek et al. (Oezipek, S., Hoelterhoff, S., Breuer, S., Bell, C., & Bathke, A. (2022). mD-UPLC-MS/MS: Next Generation of mAb Characterization by Multidimensional Ultraperformance Liquid Chromatography-Mass Spectrometry and Parallel On-Column LysC and Trypsin Digestion. Analytical Chemistry). The Oezipek system allows the analysis of a single, peptide level sample, that is processed through a novel multidimensional LC system which allowed the use of state of the art UPLC columns for the first time. The Oezipek system provides good sequence coverage and detection of small polar molecules on the peptide level within a relatively short time (~85 minutes per sample).

The combination of any three of the known methods to provide all three levels of information (native, reduced and peptide mapping) is inevitably time consuming and labour intensive even with recent advancements in peptide mapping.

The present invention aims to solve one or more of the above problems. In particular, the present invention provides a multidimensional LC system that can prepare and provide samples at multiple different levels for analysis with reduced user input. In particular, the invention aims to provide a multidimensional LC system that can prepare native, reduced and peptide mapping samples for analysis from a single sample.

### Summary of Invention

The present invention provides a multidimensional liquid chromatography (LC) system according to claim 1 for characterising a sample of antibodies such as therapeutic antibodies.

The multidimensional liquid chromatography (LC) system of the invention has:
a fractionation module having a fractionation column for providing fractionated samples
a first splitter fluidly connectable to and downstream from the fractionation module and fluidly connectable to and upstream from a reduction module wherein the first splitter has an inlet, a first outlet and a second outlet for splitting the fractionated samples into a first measurement sample, such as a native antibody measurement sample, that is passed through the first outlet and a sample for reduction that is passed through the second outlet
the reduction module having a reduction column fluidly connectable to the second outlet of the first splitter
a second splitter fluidly connectable to and downstream from the reduction module and fluidly connectable to and upstream from a digestion module wherein the second splitter has an inlet, a first outlet and a second outlet for splitting the reduced sample into a second measurement sample, such as a reduced antibody measurement sample, that is passed through the first outlet, and a sample for digestion that is passed through the second outlet
the digestion module having a digestion column containing an immobilised proteolytic enzyme for digesting the reduced sample to provide a digested sample wherein the digestion column is fluidly connectable to the second outlet of the second splitter
a separation module having a separation column for separating analytes and providing a third measurement sample, such as a peptide mapping sample.

The present invention also provides a multidimensional LC process for analysing a sample of therapeutic antibodies. The process comprises the steps of;
providing a sample of antibodies to be analysed:
introducing the sample to the system of the invention.

In some embodiments the process comprises the following steps:
and the first splitter;
at the first splitter, the sample is split into a first and second flow;
the first flow is the first measurement sample and may be flowed to an analysis module;
the second flow is flowed to the reduction module and is reduced before passing to the second splitter;
at the second splitter, the sample is split into a third and fourth flow;
the third flow is the second measurement sample and may be flowed to the analysis module; and
the fourth flow is flowed to the digested module and is digestion before being separated to provide the third measurements sample.

### Brief Description of the Figures

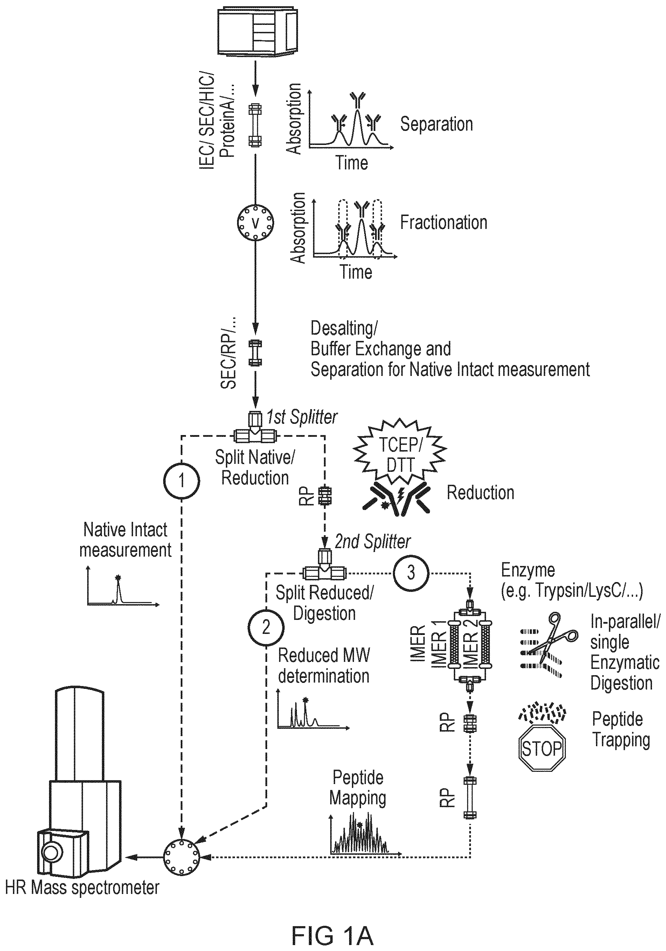

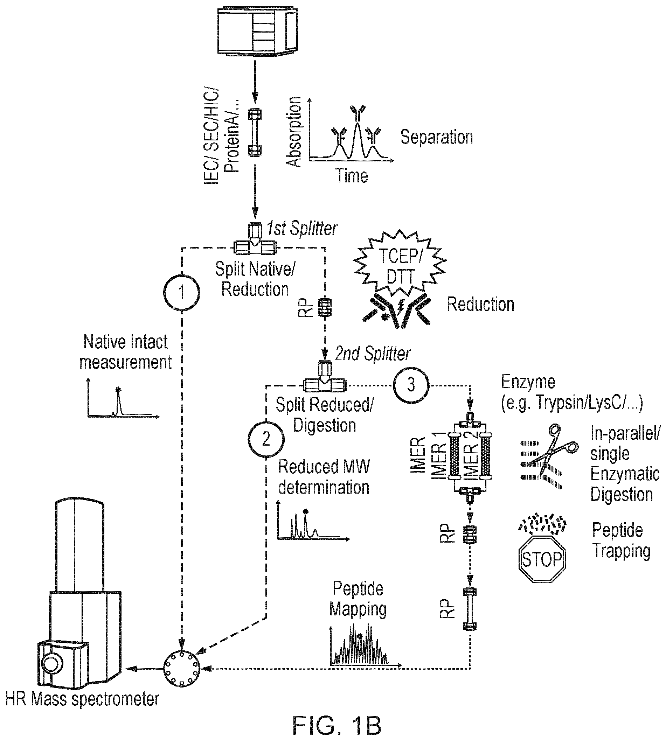

Figures 1A and 1B show schematic for two multidimensional liquid chromatography (LC) systems of the invention (often referred to "3-in 1 mD-UHPLC"). Figure 1A shows a schematic whereby the optional multiple heart cutting valve is shown after the first separation and a buffer exchange module is provided downstream from the fractionation module and upstream from the first splitter. Figure 1B shows a schematic whereby a native SEC is shown for the first separation (the optional multiple heart cutting valve is not shown) and a buffer exchange module is provided downstream from and fluidly connectable to both the first outlet of the first splitter and the first outlet of the second splitter

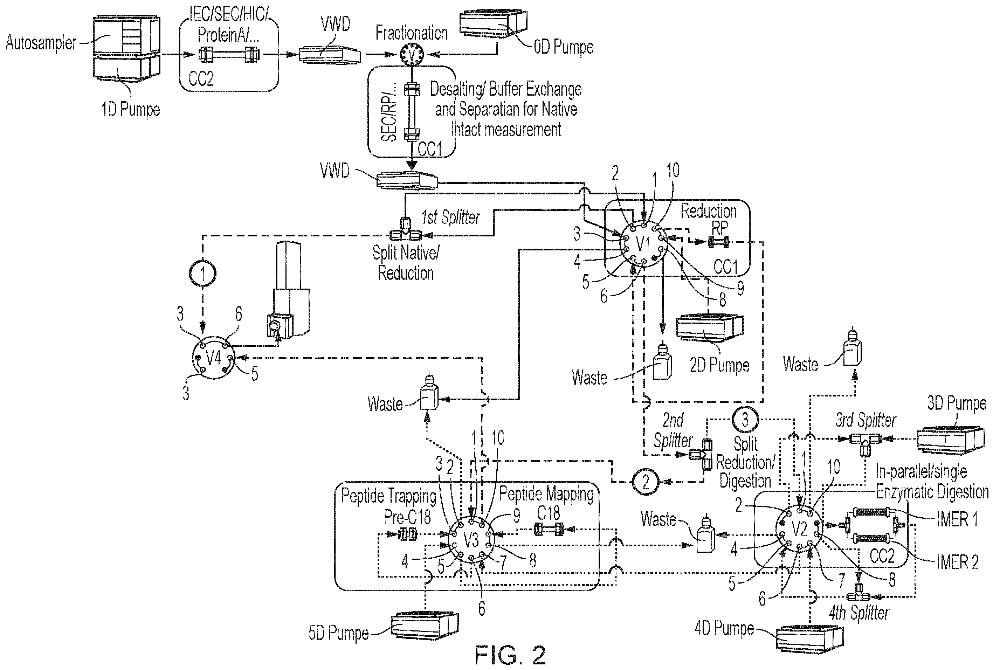

Figure 2 shows a circuit diagram of one embodiment of the mD-UHPLC/MS system of the invention. In Figure 2: "1D Pump" is the fractionation pump; "VWD" is a UV detector; "2D Pump" is a buffer exchange pump; "SEC(Nativ)" is the buffer exchange column; "V1" is the first valve assemble; "S1" is the first splitter; "W" is waste; "3D Pump" is the reduction pump; "RP" is the reduction column; "S2" is the second splitter; "V2" is the second valve assembly; "S3" is the third splitter; "4D Pump" is the digestion pump; "IMER" is the digestion column; "S4" is the fourth splitter; "5D Pump" is the trapping pump; "preC18" is the trapping column; "P6" is the separation pump; "C18" is the separation column, "V3" is the third valve assembly; "V4" is the fourth valve assembly; and "M5" is the mass spectrometer. The flow from to and from the "Pre-C18" column is reversed when V3 is switched to the alternative configuration to that shown. In this way, the trapped sample is pumped by the 5D pump off the trapping column and onto the C18 peptide mapping column.

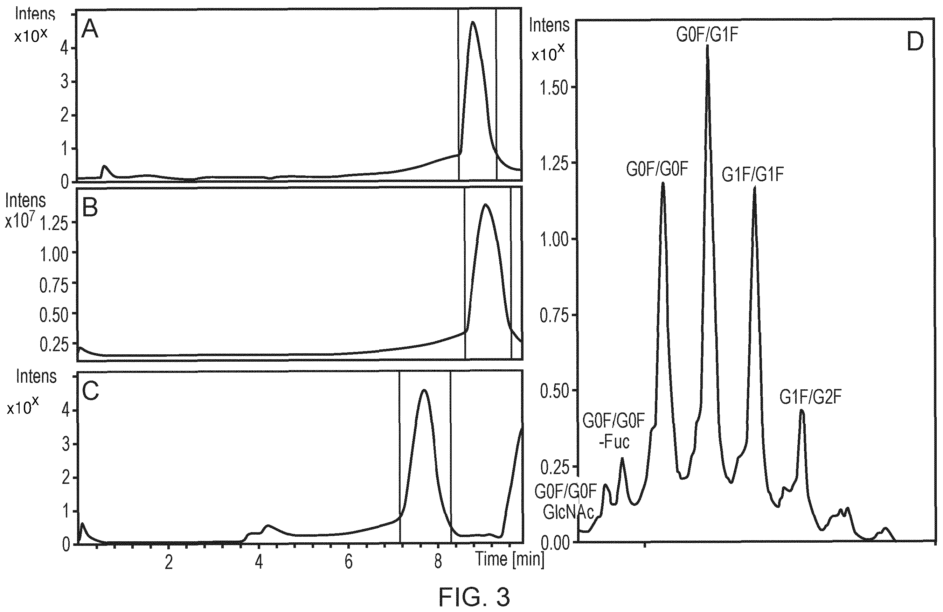

Figure 3 shows the measurements for the native mAb1 using the offline, online and 3-in-1 method. Figure 3A shows the total ion chromatogram of native offline measurement; Figure 3B show the total ion chromatogram of native online measurement; Figure 3C show the total ion chromatogram of native 3-in-1 measurement; Figure 3D shows the deconvoluted mass spectrum of offline measurement and serves as an example of the results of all methods. You can see the modifications of N-glycosylation which include G0F/G0F-GlcNAc, GOF/GOF-Fuc, GOF/GOF, G0F/G1F, G1F/G1F and G1F/G2F.

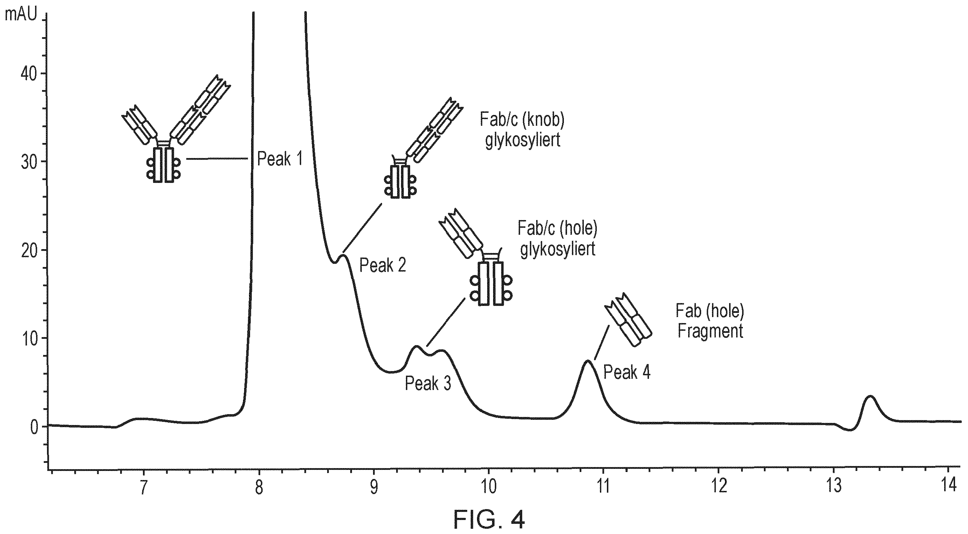

Figure 4 shows the size exclusion chromatogram of the mAb2. The size exclusion chromatogram of the mAb2 was recorded by means of a VWD at 214 nm / DAD. Peak 1 is the intact antibody, peak 2 is the fab/c (knob), peak 3 is the fab/c hole and peak 4 is the fab hole fragment.

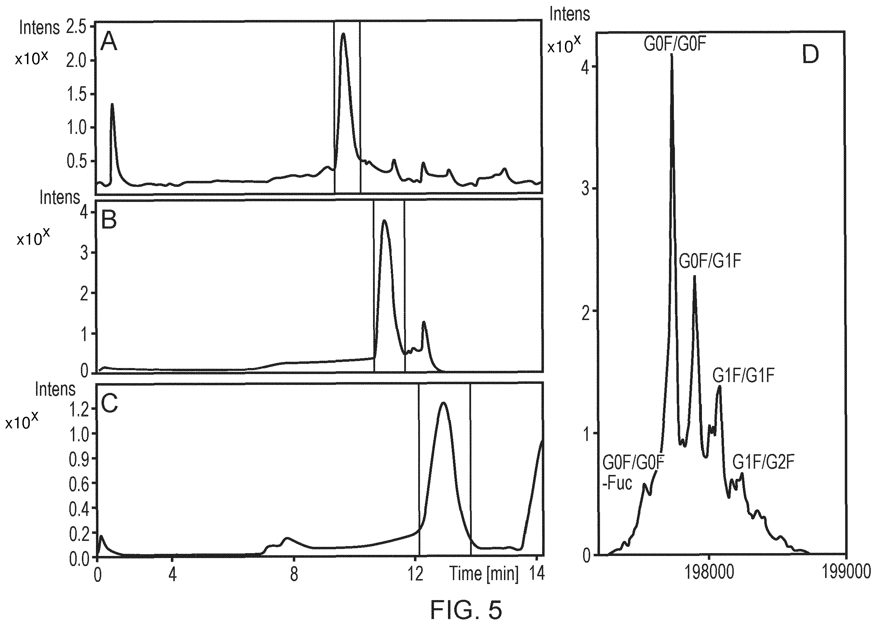

Figure 5 shows the measurements for the native mAb2 using the offline, online and 3-in-1 method. Figure 5A shows the total ion chromatogram of the native offline measurement of the mAb2; Figure 5B shows the total ion chromatogram of the native online measurement of mAb2; Figure 5C shows the total ion chromatogram the native 3 in 1 measurement of the mAb2; Figure 5D shows the deconvoluted mass spectrum of offline measurement. The modifications G0F/G0F-Fuc, GOF/GOF, G0F/G1F, G1F/G1F and G1 F/G2F can be seen.

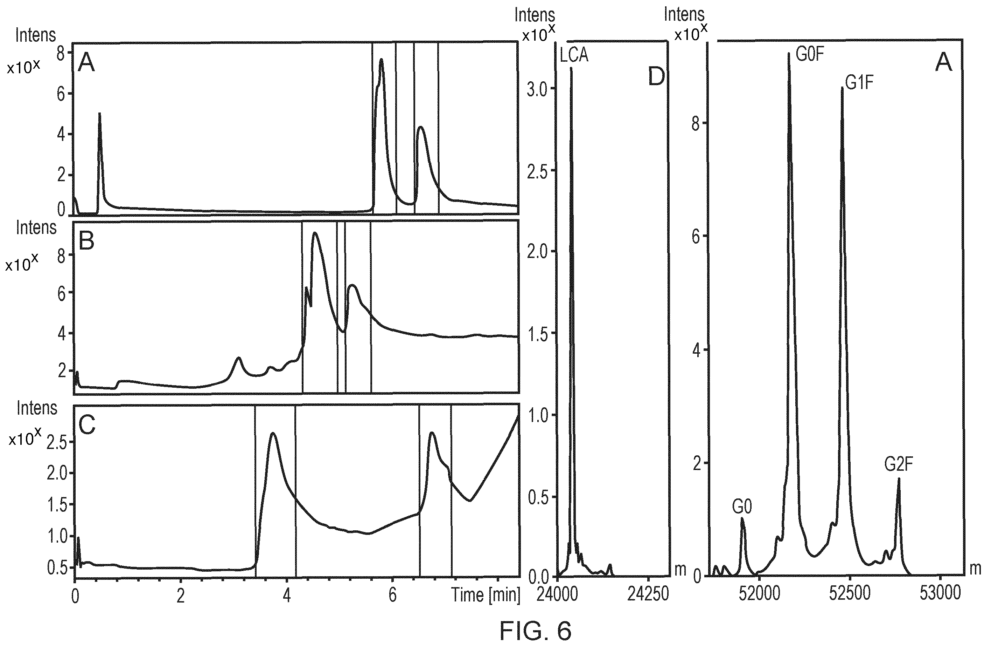

Figure 6: shows the measurements for the reduced mAb1 using the offline, online and 3-in-1 method. Figure 6A shows the total ion chromatogram of reduced offline measurement; Figure 6B show the total ion chromatogram of reduced online measurement; Figure 6C show the total ion chromatogram of reduced 3-in-1 measurement; Figure 6D shows the deconvoluted mass spectrum of the light chain of the mAb 1; Figure 6E shows the deconvoluted mass spectrum of the heavy chain of the mAb1. No modifications were discovered on the light chain, in contrast to the heavy chain on which the modification G0, G0F, G1F and G2F was found.

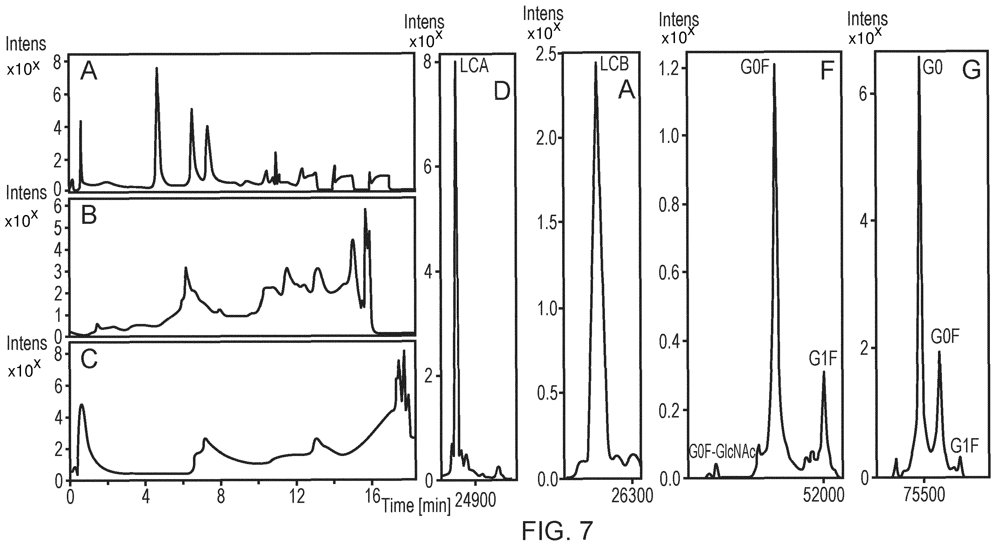

Figure 7: shows the measurements for the reduced mAb2 chains using the offline, online and 3-in-1 method. Figure 7A shows the total ion chromatogram of reduced offline measurement; Figure 7B show the total ion chromatogram of reduced online measurement; Figure 7C show the total ion chromatogram of reduced 3-in-1 measurement; Figure 7D shows the deconvoluted mass spectrum of the Light chain A; Figure 7E shows the deconvoluted mass spectrum of the Light chain B; Figure 7F shows the deconvoluted mass spectrum of the heavy chain H; Figure 7G shows the deconvoluted mass spectrum of the heavy chain K. The light chains do not carry any modifications. The heavy chain H is shown with the modifications of N-glycosylation, which would be G0F-GlcNAc, G0F and G1F. The heavy chain K is shown in with the modification G0, G0F and G1F.

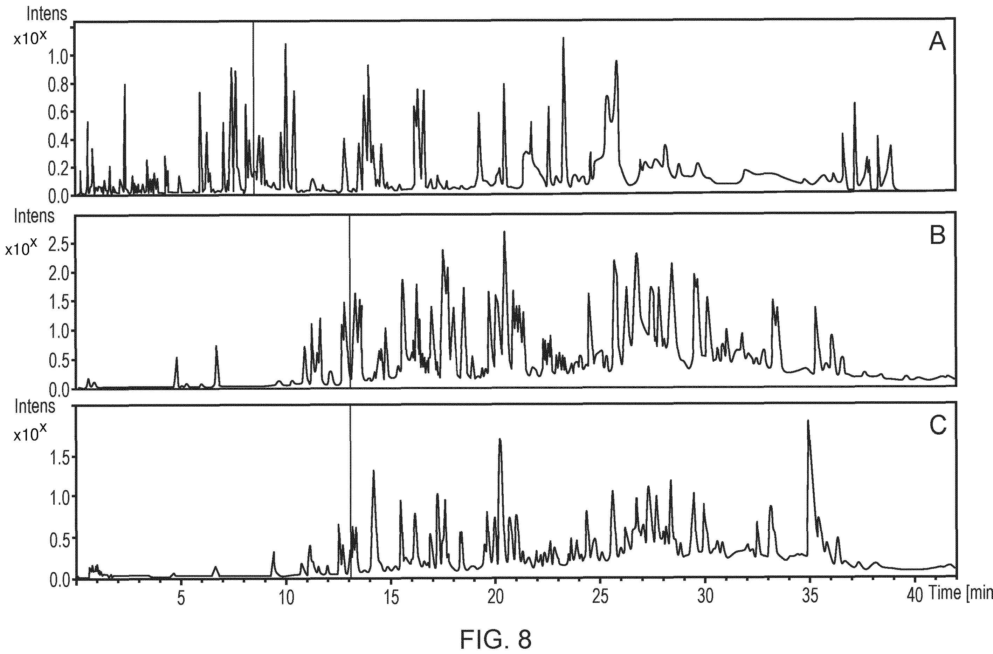

Figure 8 shows the measurement of peptide mapping of the mAb1 using the offline, online and 3 in 1 method. Figure 8A shows the total ion chromatogram of the peptide mapping of the offline method. Figure 8B shows the total ion chromatogram of the peptide mapping of the online method. Figure 8C shows the total ion chromatogram of the 3 in 1 method.

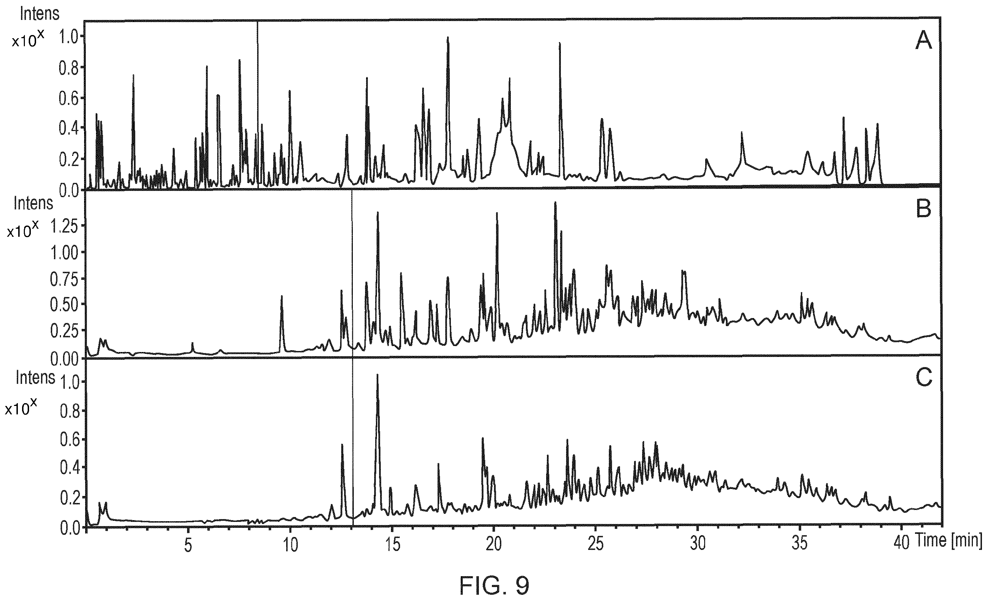

Figure 9 shows the measurement of peptide mapping of the mAb2 using the offline, online and 3 in 1 method. Figure 9A shows the total ion chromatogram of the peptide mapping of the offline method. Figure 9B shows the total ion chromatogram of the peptide mapping of the online method. Figure 9C shows the total ion chromatogram of the 3 in 1 method.

### Detailed Description

The present invention provides a multidimensional liquid chromatography (LC) system for characterising a sample of antibodies, such as therapeutic antibodies. The system provides multiple different measurement samples from a single sample, for example, the system may provide a native antibody sample for measurement, a reduced antibody sample for measurement and digested sample for peptide mapping from a single sample. The single sample may be a provide by a single injection into the multidimensional liquid chromatography (LC) system.

The multidimensional liquid chromatography (LC) system of the invention has:
a fractionation module having a fractionation column for providing fractionated samples
a first splitter fluidly connectable to and downstream from the fractionation module and fluidly connectable to and upstream from a reduction module wherein the first splitter has an inlet, a first outlet and a second outlet for splitting the fractionated samples into a first measurement sample, such as a native antibody measurement sample, that is passed through the first outlet and a sample for reduction that is passed through the second outlet
the reduction module having a reduction column fluidly connectable to the second outlet of the first splitter
a second splitter fluidly connectable to and downstream from the reduction module and fluidly connectable to and upstream from a digestion module wherein the second splitter has an inlet, a first outlet and a second outlet for splitting the reduced sample into a second measurement sample, such as a reduced antibody measurement sample, that is passed through the first outlet, and a sample for digestion that is passed through the second outlet
the digestion module having a digestion column containing an immobilised proteolytic enzyme for digesting the reduced sample to provide a digested sample wherein the digestion column is fluidly connectable to the second outlet of the second splitter
a separation module having a separation column for separating analytes in the digested sample and providing a third measurement sample, such as a peptide mapping sample.

In this way, the system of the invention provides measurement samples for characterization of a samples, such as antibodies, on multiple different levels. In particular, the system allows analysis of antibodies on three different protein levels; the native level (the 'first measurement sample'); the reduced level ('the second measurement sample); and the peptide level (the 'third measurement sample').

In use, the sample for reduction from the first splitter may be loaded onto the reduction column and reduced whilst the first measurement sample is analysed, such as by mass spectrometry. Similarly, the sample for digestion from the second splitter may be loaded onto the digestion column and digested whilst the second measurement sample is analysed, such as by mass spectrometry. The third measurement sample is subsequently produced from the separation module after digestion (and after the second sample has been analysed) and can be analysed, such as by mass spectrometry. In this way, the system of the invention can process three different measurement samples sequentially and in a time efficient manner.

Surprisingly, the system of the invention provides comparable results for each of the different samples measured compared to the known, separate measurement methods. The system provides a fast and efficient method for antibody characterization on multiple levels.

The system of the invention also provides the multiple measurements from a single injected sample. This significantly reduces the hands-on time for obtaining the multiple measurements compared to the known, separate measurement methods. This also provides the benefit of reducing user errors such as sample contamination or mixing of samples when using the known, separate measurement methods.

The multidimensional liquid chromatography (LC) system of the invention may also preferably have a buffer exchange module having a buffer exchange column. the buffer exchange column may be a size exclusion chromatography column or a reverse phase chromatography column.

The buffer exchange module may be downstream from to the fractionation module and upstream from and fluidly connectable to the first splitter. In some cases, the buffer exchange module may fluidly connectable to the fractionation module.

In this way, the first and second measurement samples are desalted and buffer exchanged before analysis. It is proposed that the lower salt level results in less contamination of the samples and subsequently less contamination of the analysis system used such as a mass spectrometer. The resulting analysis of each of the measurement samples may be more robust.

In some embodiments, the multidimensional LC system of the invention further comprising an analysis module for analysing the first, second or third measurement. The analysis module is fluidly connectable to the first outlet of the first splitter, the first outlet of the second splitter and the separation column. The analysis module may comprise: a mass spectrometer such as a high-resolution mass spectrometer (HRMS) or a Single Quad mass spectrometer; an evaporative light scattering detector (ELSD): a UV detector; or a diode array detector (DAD). In some embodiments, the first, second and third measurement samples are analysed by the same analysis module, preferably in such cases the analysis module comprises a mass spectrometer such as a high resolution ESI mass spectrometer.

In some embodiments, the multidimensional LC system of the invention further comprising and injection module for injecting the sample in the system. The injection module may comprise an autosampler, or a direct injector. In some embodiments, the injection module may further comprise a bioreactor for preparing the sample to be analysed. In some embodiments, the injection module is part of the fractionation module. For example, the injection module and the fractionation module combined may be a PAT fractionation module or an autosampler fractionation module.

In some embodiments, the multidimensional LC system further comprises at least one column oven, preferably the multidimensional LC system comprises three column ovens. In this way, the temperature of one or more of the components can be controlled.

### Definitions

A "valve assembly" refers to a multi-port valve component that controls flow between elements connected to the ports. This is typically achieved by a switch mechanism that moves one or more valve conduits to switch communication between different elements. Elements, e.g. components of one or more of the modules, may be fluidically connected to the ports via further conduits, like pipes, tubes, capillaries, microfluidic channels and the like and by fittings like screws/nuts and ferrules, or alternative liquid-tight sealings, e.g., maintained in place by a clamp mechanism. In this way, the various components of the modules may be connected as defined herein. Or example, the pump of the trapping module may be connected to the trapping column via the third valve assembly.

"Liquid chromatography" or "LC" is an analytical process that subjects sample to chromatographic separation through an LC column in order, for example, to separate analytes of interest from matrix components. Samples may be injected by a sample injector.

"High-performance liquid chromatography" or HPLC, "ultra-high-performance liquid chromatography" or UHPLC, including "micro liquid chromatography" or µLC and "small-bore liquid chromatography" or small-bore LC are forms of liquid chromatography performed under pressure.

A "liquid chromatographic system or LC system" is an analytical apparatus or a unit in an analytical apparatus for carrying out liquid chromatography. The LC system may also comprise elements such as a sample injector, valves, liquid sources, fluidic connections e.g., for mixing liquids, degassing liquids, tempering liquids, and the like, one or more sensors, such as pressure sensors, temperature sensors and the like, and especially at least one LC pump. The list is not exhaustive. According to an embodiment, the LC system of the invention is an analytical apparatus designed to prepare multiple samples for mass spectrometry and/or to transfer a prepared sample to a mass spectrometer, in particular for separating analytes of interest before detection by a mass spectrometer.

A "multidimensional LC system" refers to an LC system as defined herein in which multiple modules are combined. Each module is referred to as a dimension. Each module contains a column through which the sample is passed. Different types of columns can be used in different modules to provide a sequence of chromatography and treatment, e.g. reduction or digestion, steps in a single 'run' through the LC system. Each module may also comprise components such as a pump and waste outflow. Generally, multidimensional LC systems also have a number of valve assemblies for connecting the different modules at different times.

A "column" refers to any of a column, a cartridge, a capillary and the like that are suitable for performing chromatography or performing a reaction such as digestion on a substance that is passed through the column. Preferably the column is a column. Columns are typically packed or loaded with a stationary phase, through which a mobile phase is pumped in order to trap and/or separate and elute and/or transfer analytes of interest under selected conditions, e.g., according to their polarity or log P value, size or affinity, as generally known. This stationary phase can be particulate or beadlike or a porous monolith. Columns may be exchangeable and/or operate in parallel or in sequence to one or more other columns.

The term "sample" refers to a material suspected of containing one or more analytes of interest. In the present case, the sample is preferably a sample of antibodies for analysis in particular therapeutic monoclonal antibodies. The sample can be pre-treated prior to use. Methods of treatment can involve filtration, centrifugation, distillation, concentration, inactivation of interfering components, and the addition of reagents.

Some features of the system of the invention are defined in terms of their position relative to other components in the direction of flow (e.g. upstream or downstream). The direction of flow in this case refers to the flow of liquid, e.g., solvent, through the system during operation. For example, if component A is 'upstream' from component B, the liquid, e.g. solvent, will pass through component A before component B during operation of the system. Similarly, if component C is 'downstream' from component D, the liquid, e.g., solvent, will pass through component D before component C during operation of the system.

### Fractionation Module

The multidimensional LC system comprises a fractionation module having a fractionation column for providing fractioned samples. The fractionation column is a chromatography column for separating components in the sample to be analysed.

In this way, the system of the invention can separate an initial sample into fractions each of which can then be further processed in the other modules.

In some embodiments, the fractionation column is selected from an ion exchange chromatography column such as a cation exchange chromatography column, size exclusion chromatography column, a hydrophilic interaction chromatography column (HILIC), a hydrophobic interaction chromatography column (HIC) or a proteinA affinity column. Preferably the fractionation column is a size exclusion chromatography column or an ion exchange chromatography column.

The fractionation column may be selected to provide the required analysis of the sample, for example, where size variants in a sample are of interest a size exclusion chromatography column may be used to separate out components by size. The fractionation column may be selected to correspond with the GxP method for quality control analysis of the mAb to be analysed.

In some embodiments, the multidimensional LC system further comprises a multiple heart cutting valve.

The multiple heart cutting valve may be fluidly connected to the fractionation column. The term "multiple heart cutting valve" refers to a valve assembly that allows for the online fractionation and in loop storage of fractions in a liquid chromatography system. Examples of multi heart cutting valves include a 2 position 4 port duo valve from Agilent technologies (product no. G4236A) which may be couple with one or two loop decks such as a 6 position 14 port valve loop deck from Agilent technologies (product no. G4242A).

The loop storage of the multiple heart cutting valve may be from 40 to 150 µL. In some embodiments the loop storage may be from 100 to 150 µL. Larger loop storage means more of a fraction can be retained for further processing and analysis resulting in more of the analyte and the detection of less common fragments.

The multiple heart cutting valve may be after the fractionation column in the direction of flow. In some such embodiments, the multiple heart cutting valve is also fluidly connected to the reduction module via the first splitter.

In some embodiments the fractionation module comprises a fractionation pump and the fractionation pump is fluidly connected to the fractionation column. The fractionation pump may be a binary or quaternary pump.

If a multiple heat cutting valve is not used in the LC system of the invention, fractionation is also feasible with common valve-loop combinations.

### Buffer Exchange Module

The multidimensional liquid chromatography (LC) system of the invention may also have a buffer exchange module having a buffer exchange column. The buffer exchange column may be a size exclusion chromatography column or a reverse phase chromatography column. Preferably the buffer exchange column is a size exclusion chromatography column.

The buffer exchange module may be downstream from to the fractionation module and upstream from and fluidly connectable to the first splitter. In some cases, the buffer exchange module may fluidly connectable to the fractionation module.

In this way, the first and second measurement samples are desalted and buffer exchanged before analysis. It is proposed that the lower salt level results in less contamination of the samples and the analysis system used such as a mass spectrometer. The resulting analysis of each of the measurement samples may be more robust.

Preferably, the size exclusion chromatography column of the buffer exchange modules is a biocompatible SEC column with low interactions between the analyte and the column material. In some embodiments, the size exclusion chromatography column contains a stationary phase comprising ethylene bridged hybrid particles such as an ACQUITY Premier Protein SEC Column with MaxPeak Premier SEC.

In some cases, the buffer exchange column has a length of from 100 to 400 mm, preferably from 200 to 350 mm such as around 300 mm.

In some cases, the buffer exchange column has a packing material with average particle size of 1 to 10 µm, preferably from 1.5 to 2.0 µm such as around 1.7 µm. Average particle size may be measured using dynamic light scattering or sieve analysis.

In some cases, the buffer exchange column has an internal diameter of 3 to 10 mm, preferably from 4 to 7mm such as around 4.6 mm.

In some cases, the buffer exchange column has an average pore size of from around 100 Å to 350 Å, preferably from 200 Å to 300 Å such as around 250Å. The pore size may be measured by gas adsorption for example using the Brunauer Emmett Teller theory.

Preferably an analysis module is provided downstream from the buffer exchange module. The analysis module is fluidly connectable to the first outlet of the first splitter, the first outlet of the second splitter and the separation column. The analysis module may be connectable to the first outlet of the first splitter and the first outlet of the second splitter via the buffer exchange module. The analysis module may comprise a mass spectrometer.

### First Splitter

The multidimensional LC system has a first splitter. The first splitter is fluidly connectable to and downstream from the fractionation module. The first splitter is fluidly connectable to and upstream from the reduction module. The first splitter has an inlet, a first outlet and a second outlet. In use, the first splitter splits the fractionated samples into a first measurement sample, such as a native antibody measurement sample, that is passed through the first outlet and a sample for reduction that is passed through the second outlet.

In some embodiments the first outlet of the first splitter is fluidly connectable to an analysis module.

In some embodiments the first splitter is adjustable to adjust the split the of the flow between the first outlet : second outlet. In some cases, the flow may be split in a ratio of from 2:98 to 35 :65, preferably from 10:90 to 4: 96. The ratio of the split may be controlled by adjusting the grit ratio of the first splitter.

In this way, the larger portion of the flow is sent to the reduction column for reduction whilst a large enough portion is split into the first measurement sample to allow the native measurement to be carried out. The ratio of the split (and the initial sample concentration) may be varied to ensure sufficient analyte is present in the measurements samples to provide accurate measurements.

### Reduction Module

The multidimensional LC system has a reduction module having a reduction column. The reduction module is upstream from the second splitter and downstream from the first splitter.

In this way, the fractionated sample can be reduced before passing into the second splitter.

The reduction column is fluidly connectable to the second outlet of the first splitter, preferably the reduction module is fluidly connected to the second outlet of the first splitter. The sample is introduced on the reduction column and reduced for example by passing a solution of reducing agent through the reduction column to reduce the sample on the column.

In some embodiment, the reduction column contains a C3 or C4 stationary phase, preferably a C4 stationary phase. In this way, the column provides a balance between retention so that reduction can be performed and elution to remove the reduced sample for further processing in the system. Suitable commercially available columns for the reduction column include the Waters ACQUITY UHPLC Protein BEH C4 VanGuard Pre-column, 300Å, 1.7 µm, 2.1 mm X 5 mm, 10K - 500K or the Agilent Technologies PLRP-S 1000A 5um, 2.1x50mm PEEK lined.

In some cases, the reduction column has a length of from 10 to 200 mm, preferably from 20 to 100 mm such as around 50 mm.

In some cases, the reduction column has a packing material with average particle size of 1 to 10 µm, preferably from 4 to 6 µm such as around 5 µm. Average particle size may be measured using dynamic light scattering or sieve analysis.

In some cases, the reduction column has an internal diameter of 0.5 to 5 mm, preferably from 1 to 3 mm such as around 2.1 mm.

In some cases, the reduction column has an average pore size of from around 500 Å to 2000 Å, preferably from 900 Å to 1400 Å such as around 1000Å. The pore size may be measured by gas adsorption for example using the Brunauer Emmett Teller theory.

In some embodiments the reduction module comprises a reduction pump and the reduction pump is fluidly connectable to the reduction column. In embodiments having a multiple heart cutting valve, the multiple heart cutting valve may be between the reduction pump and the reduction column in the direction of flow. The reduction pump may be a binary or quaternary pump. Preferably, the reduction pump of the reduction module is a quaternary pump.

### Second Splitter

The multidimensional LC system has a second splitter. The second splitter is fluidly connectable to and downstream from the reduction module. The second splitter is fluidly connectable to and upstream from the digestion module. The second splitter has an inlet, a first outlet and a second outlet. In use, the second splitter splits the reduced sample into a second measurement sample, such as a reduced antibody measurement sample, that is passed through the first outlet and a sample for digestion that is passed through the second outlet to the digestion module.

In some embodiments the first outlet of the second splitter is fluidly connectable to an analysis module.

In some embodiments the second splitter is adjustable to adjust the split the of the flow between the first outlet : second outlet. In some cases, the flow may be split in a ratio of from 2:98 to 35 :65, preferably from 10:90 to 4: 96. The ratio of the split may be controlled by adjusting the grit ratio of the second splitter.

In this way, the larger portion of the flow is sent to the digestion column for digestion and peptide mapping whilst a large enough portion is split into the second measurement sample to allow the reduced measurement to be carried out. The ratio of the split (and the initial sample concentration) may be varied to ensure sufficient analyte is present in the measurements samples to provide accurate measurements.

The ratio of the split may also be controlled in use by adjusting the flow rate through the splitter.

### Digestion Module

The multidimensional LC system comprises a digestion module having a digestion column containing an immobilised proteolytic enzyme for digesting the reduced sample to provide a digested sample. The digestion module is downstream from the second splitter and is fluidly connectable to the second outlet of the second splitter.

In some embodiments, the digestion column is selected from a Trypsin immobilized enzyme reactor or a LysC immobilized enzyme reactor.

In some embodiments, the digestion module has a first mixer such as a static mixer or a zero delay volume T-Piece downstream from the digestion column(s) in the direction of flow.

In some embodiments, the digestion module has a second mixer such as static mixer or a zero delay volume T-Piece. In some cases the second mixer is before, or upstream from, the digestion column in the direction of flow such that the sample passes through the mixer and is mixed for example with additional solvents to provide a homogenous mixture before entering the digestion column.

In some preferred embodiments, the digestion module comprises two digestion columns. In some such cases the two digestion columns are connected in parallel such that in use the sample flow is split between the two columns.

In some such cases, the two digestion columns are independently selected from a Trypsin immobilized enzyme reactor and a LysC immobilized enzyme reactor, preferably the two digestion columns are a Trypsin immobilized enzyme reactor and a LysC immobilized enzyme reactor.

The parallel digestion setup provides unique peptide combinations for example a unique Trypsin and a LysC peptide can be received. In this way, there is an increased the likelihood of post translational modification (PTM) characterization and the sequence coverage is increased. That is, using two digestion columns can provide a broader range of digestion products and enable greater sequence coverage. This in-parallel digestion may be particularly advantageous with the increasing number of more complex bispecific mAbs.

In some embodiments the digestion module comprises a digestion pump. The digestion pump may be a binary or quaternary pump.

### Separation Module

The multidimensional LC system comprises a separation module having a separation column for separating analytes and providing a third measurement sample, such as a peptide mapping sample.

The separation module is configured to receive the digested sample from the digestion column. In some embodiments, the separation column is not fluidly connectable to the digestion column directly. In such cases, the transfer of the digested sample takes place via another module, such as a trapping module.

In some embodiments the separation module comprises a separation pump. The separation pump may be a binary or quaternary pump.

In some embodiments the separation column is selected from a peptide mapping column such a UHPLC column, a HPLC column, a reverse phase chromatography column, or a hydrophilic interaction chromatography column, preferably, the separation column is a UHPLC column. Preferably, the separation column is a UHPLC column.

In some cases, the separation column has a C18 stationary phase. C18 stationary phases are typically used for chromatographic separation of peptides. In this way, good peptide retention and separation can be achieved.

In some cases, the separation column has a length of 100 to 200 mm, such as around 150 mm.

In some cases, the separation column has a packing material with particle size of 1.0 to 3.0 µm, for example, 1.0 to 2.0 µm, preferably 1.5 to 2.0 µm.

In some cases, the separation column has an internal diameter of 1.5 to 5 mm, preferably 1.5 to 2.5 mm such as around 2.1 mm.

In some cases, the separation column contains a C18 stationary phase.

### Trapping Module

The multidimensional LC system of the invention may further comprise a trapping module having a trapping column for trapping the digested sample. The trapping module is downstream from the digestion module and upstream from the separation module.

The trapping column is selected to have a stationary phase that retains the analytes of interest, e.g. digested antibodies, whereas any salts, buffer, detergents and other matrix components are unretained and can be washed away.

The trapping column allows the de-coupling of the digestion column and the separation column. The trapping column may also serve to protect the separation column by trapping unwanted components e.g. indigested proteins in the case of protein mapping.

In some embodiments the trapping column has a length of 5 to 30 mm. In this way, the trapping column allows trapping of the sample and dilution of acetonitrile without significantly increasing the backpressure to the digestion column.

In some cases the trapping column has a length of from 5 to 10mm. In this way, sufficient trapping occurs and the back pressure can be reduced.

In some cases the trapping column has a length of from 25 to 30 mm. In this way, increased trapping occurs which is useful particularly for cases where trapping performance is very important.

In some embodiments the trapping column has a packing material with particle size of 1.0 to 3.0 µm, for example 1.0 to 2.0 µm preferably 1.5 to 2.0 µm.

In some embodiments the trapping column has an internal diameter of 1.5 to 5 mm. In some cases, the internal diameter is from 2.5 to 4.7 mm.

In some embodiments the trapping column has the same packing material as the peptide mapping column. Preferably, the trapping column has a C18 stationary phase.

In some embodiments the trapping module comprises a trapping pump. The trapping pump may be a binary or quaternary pump.

### Valve Assemblies

The multidimensional LC system of the invention may further comprise at least one valve assembly. The valve assemblies may be configured to connect and dis-connect two or more of the modules during operation.

The valve assemblies may be any a multiport valve with from a 2 to 7 way switching, preferably 2 way switching.

The valve assemblies may be a multiport valve with any of 6, 10, 12, or 14 port valves. Preferably the valve assemblies are 6 or 10 port valves with 2 way switching.

In some embodiments, the system has a first valve assembly configured so that in a first position the inlet of the first splitter is fluidly connected to the fractionation module and, when present, the buffer exchange module or the multiple heart cutting valve. In the first position of the first valve assembly the first outlet of the first splitter is fluidly connected to another module of the system such that a first measurement sample can be split from the flow and the second outlet of the first splitter is fluidly connected to the reduction module. The another module of the system may be an analysis module. In this way, the fractionated sample can be sent for analysis and reduction. The first valve assembly is configured so that in a second position the inlet and the second outlet of the first splitter are connected in a loop. In the second position of the first valve assembly the reduction module is fluidly connected to the second splitter and when present the reduction pump. In the second position, there is no effective flow through the first splitter and the reduction module is not connected to the splitter allowing reduction to occur on the sample loaded on to the column.

In some embodiments, the system has a second valve assembly configured so that in a first position the second outlet of the second splitter is fluidly connected to the digestion module and, when present, the trapping module. In this way, reduced sample can be loaded onto the digestion column for digestion and may be trapped on the trapping column when present.

In the first position of the second valve, the second outlet of the second splitter may be fluidly connected to the digestion module via a third splitter having two inlets, one for the sample flow and one for the digestion pump. In this way, the digestion pump when present can be used to dilute the sample for digestion to an appropriate concentration and buffer.

In the first position of the second valve, the digestion column may be fluidly connect to the trapping module when present by a fourth splitter having two inlets, one for the digested sample flow and one for the trapping pump to dilute the digested sample for trapping. In this way, the solvent concentration after digestion can be adjusted to provide a solvent system that promotes trapping on the trapping column.

In a second position of the second valve assembly, the digestion module is isolated and is not in fluid connected with any other components for example, the digestion module is not fluidly connected to the trapping module when present or the reduction module.

In some embodiments, the system has a third valve assembly configured so that in a first position the trapping module is fluidly connected to the digestion module and is not fluidly connected to the separation module. In the second position of the third valve assembly the trapping module is fluidly connected to the separation module and is not fluidly connected to the digestion module. In this way, the digestion module is uncoupled from the separation module and the different pressures that may preferably be used in these two different modules are isolated from each other.

In some embodiments, the flow through the trapping column may be reversed when the third valve assembly is switched from the first to the second position.

In some embodiments, the system has a fourth valve assembly configured so that in a first position the first outlet of the first splitter is fluidly connected to another module of the system and so that in a second position the first outlet of the second splitter is fluidly connected to another module of the system. The another module may be an analysis module such as a mass spectrometer. The first outlet of the second splitter may be connected to the another module of the system via the third valve assembly when present for example when the third valve assembly is in the first position. The separation module may be fluidly connected to the another module of the system when the third valve assembly is in the second position and the fourth valve assembly is in the second position. In this way, the third and fourth valve assemblies can be used to control which of the first, second or third measurement sample is sent for analysis for example to an analysis module.

The multidimensional LC system of the invention may comprise and combination of the first, second, third and fourth valve assemblies discussed above. Preferably the multidimensional LC system of the invention has a first, a second, a third and a fourth valve assembly as described above and in combination with the other optional features discussed for each valve assembly.

### Other

Other aspects and embodiments of the invention provide the aspects and embodiments described above with the term "comprising" replaced by the term "consisting of" and the aspects and embodiments described above with the term "comprising" replaced by the term "consisting essentially of".

Modifications of the above embodiments, further embodiments and modifications thereof will be apparent to the skilled person on reading this disclosure, and as such, these are within the scope of the present invention, insofar as they come within the scope of the enclosed claims.

"and/or" where used herein is to be taken as specific disclosure of each of the two specified features or components with or without the other. For example, "A and/or B" is to be taken as specific disclosure of each of (i) A, (ii) B and (iii) A and B, just as if each is set out individually herein.

### Process

The present invention also provides a multidimensional LC process according to claim 12 for analysing a sample of therapeutic antibodies.

The process is carried out using a system of the invention. The terminology provided for the system above is used below to define the process. Optional and preferred features of the system may be implemented in the process of the invention.

The process of the invention involves the steps of:
providing a sample of antibodies to be analysed:
introducing the sample to the system of the invention.

The sample is flowed through the fractionation module and the first splitter. At the first splitter, the sample is split into a first and second flow. The first flow is the first measurement sample and may be flowed to an analysis module. The second flow is flowed to the reduction module and is reduced before passing to the second splitter. At the second splitter, the sample is split into a third and fourth flow. The third flow is the second measurement sample and may be flower to the analysis module. The fourth flow is flowed to the digested module and is digestion before being separated to provide the third measurements sample.

The sample is flowed through the system using solvent and one or more pumps.

The valve assemblies (discussed above) are used to control the flow of the sample through the system and provide parallel sample measurements.

In the process a first, second, third and fourth valve assembly may be used to control when each of the first, second and third measurement samples are sent for analysis for example to an analysis module. The valve assemblies may also be used to effectively uncouple the digestion column and the separation column.

In some cases, the process of the invention comprises the following steps in order:
1 . The sample is introduced to the system and fractionated by the fractionation module, the fractions are optionally stored in a multiple heart cutting valve
2. Optionally, the fractions are flowed through a digestion module to the first splitter
3. The first valve assembly is in the first position, the second valve assembly is in the first position and the fourth valve assembly is in the first position so the first measurement sample is separated and analyzed and the rest of the sample is loaded onto the reduction column.
4. The first valve assembly is switched to the second position and the sample is reduced
5. After the sample is reduced, the second valve is switched to the second position and the fourth valve assembly is switched to the second position and the third valve assembly is in the first position
6. The reduced sample is flushed off the reduction column to the second splitter so the second measurement sample can be separated and passed through the third and fourth valve assemblies to be analyzed and the rest of the reduced sample is passed through the digestion column to a trapping column if present or directly to the separation column if no trapping column is present.
7. If the trapping column is present, the third valve assembly is in the first position when the digested sample is trapped and is then switched to the second position so that the digested sample can be passed to the separation column.

The reduction step may be carried out by introducing the sample into a reduction module comprising a reduction column and reducing the sample by flowing a solvent containing a reduction agent through the reduction column whilst the sample is on the reduction column. Preferably, the reducing agent is Tris-(2-carboxyethyl)-phosphin or dithiothreitol (DTT).

In some embodiments, the fractionation module contains a fractionation pump. The fractionation pump may pump solvent through the fractionation column to the multi heart cutting valve when present.

In some embodiments, the reduction module contains a reduction pump. The reduction pump may pump solvent comprising a reducing agent such as Tris-(2-carboxyethyl)-phosphin or Dithiothreitol through the reduction column to reduce a sample on the reduction column.

In some embodiments, the digestion module contains a digestion pump. The digestion pump may pump solvent containing the digestion buffer onto the digestion column. For example, the digestion pump may be connected to the system by a third splitter.

In some embodiments, the separation module contains a separation pump. The separation pump may pump solvent through the separation column. When the third valve assembly is in the second position, the separation pump pumps solvent through the trapping column and then through the separation column.

In some embodiments, the process further comprises the step of analysing the fractions of the sample as they flow off the separation column by mass spectrometry.

In some embodiments, where a trapping module is present, the trapping module contains a trapping pump. The trapping pump may pump solvent through the trapping column. When the second valve assembly is in the second position and the system has a fourth splitter, the trapping pump may pump solvent through the fourth splitter to dilute the solvent mixture that comes off the digestion column.

In this way, the trapping pump of the trapping module is used to adjust the solvent whilst the sample is trapped on the trapping column, for example when the solvent is a water acetonitrile mixture, the acetonitrile concentration is adjusted to from 1 to 5 wt%. Preferably, the digestion module has a fourth splitter that is after the digestion column in the direction of flow. The trapping pump is fluidly connected to the fourth splitter when the second valve assembly is in the second position. In this position, the trapping pump can pump solvent into the fourth splitter to adjust the solvent of the sample before it is loaded onto the trapping column.

In some such embodiments, the trapping pump provides a flow rate of from 0.20 to 2.5 mL/min.

This step can be used to change the solvent or liquid carrier (i.e. the mobile phase) to a preferred composition for use in the separation column. For example, in some cases, acetonitrile concentration from the digestion step is higher than preferred for the separation step. This concentration can be reduced using the isolated trapping module before passing the sample onto the separation module. This can improve the outcome of separation e.g. peptide mapping.

Preferably, the solvent used to pass the sample through the separation column is water with acetonitrile, preferably the acetonitrile concentration is from 1 to 5 wt%.

In some embodiments, the trapping column has a column temperature of from 20 to 70 °C, preferably from 30 to 50 °C.

In some embodiments, the trapping column and the separation column are at the same temperature such as from 30 to 50 °C when the digested sample is released from the trapping column and introduced onto the separation column. In this way, the separation of the sample can be optimised.

In some cases, the temperature of the trapping column will be changed between trapping of the digested sample and release of the digested sample. For example, trapping of the digested sample may be carried out at a lower temperature than release of the trapped sample. In this way trapping can be optimised without impacting significantly on separation. Preferably, during trapping the trapping column has a temperature of around 30 °C.

### Examples

In this application, a novel multidimensional LC system is provided that allows measurements of three levels of a sample, such as the native (i.e. intact), reduced and peptide level measurements of a single mAb sample from a single injection within one LC system in parallel.

The novel system set up allows all three measurements in parallel to be performed from a single injected sample during a single run through of the system.

The Examples demonstrate that the system of the invention provides comparable results to the existing separate online and offline measurements of native, reduced and peptide mapping of mAbs. The system provides these results in a fraction of the time and with significantly reduced user interaction.

The term 3-in-1 mD UHPLC MS/MS used in the application and in particular the examples refers to the system of the invention and is also shorted to "3-in-1 method". In the Examples the system is the specific system used in the experiments as outlined in Figure 1A and in more detail in Figure 2.

### Materials and Methods

### Reagents

The following reagents were using in the examples:

| **Reagent** | **Manufacturer** | **CAS No.** |
|---|---|---|
| Sodium dihydrogen phosphate monohydrate | MERCK KGaA, Darmstadt, Germany | 10049-21-S |
| di-Sodium hydrogen phosphate | MERCK KGaA, Darmstadt, Germany | 7558-79-4 |
| Calcium chloride anhydrous Powder | MERCK KGaA, Darmstadt, Germany | 10043-52-4 |
| Tris(hydroxymethyl) aminomethane (TRIS) | MERCK KGaA, Darmstadt, Germany | 77-86-1 |
| Tris(2-carhoxyethyl)phosphin (TCEP) | MERCK KGaA, Darmstadt, Germany | 51805-45-9 |
| **DL-Dithiothreitol (DTT)** | **Sigma Aldrich,** | **3483-12-3** |
| Acetontrile (ACN, HPLC-grade) | MERCK KGaA, Darmstadt, Germany | 75-05-8 |
| Sodium chloride | Fluka Missouri, USA | 7647-14-S |
| Formic acid (FA) | Fluka Missouri, USA | 64-18-6 |
| N,N-Bis(2-hydroxyethyl)-2-aminoethanesulfonic acid (BES) | MERCK KGaA, Darmstadt, Germany | 10191-18-1 |
| N, N-Bis(2-hydroxyethyl)-2-aminoethanesulfonic acid sodium salt (BES sodium salt) | MERCK KGaA, Darmstadt, Germany | 66992-27-6 |
| Purified Water (Milli-QH2O) | Merck Millipore (Merck KGaA, Darmstadt, Germany) Milli-QAdvantage A10 system | |

### Monoclonal Antibodies

Two high-purity reference standards of monoclonal antibodies designated mAb1 (F. Hoffmann-La Roche) and *mAb2* (F. Hoffmann-La Roche) were used in the experiments. mAb1 is a standard humanized monoclonal IgG antibody with two identical heavy chains (HCs) and light chains (LCs). Enzymatic digestion of mAb1 with trypsin results in 20 peptides for the LC and 42 peptides for the HC. The numbering for each mAb1 chain starts at its N-terminus and is marked with a «T» for the protease trypsin (mAb1= LC: T1-T20; HC: T1-T42).

mAb2 is a two+one bispecific antibody that has two humanized Fab arms and an additional Fab arm that is crosslinked to one of the humanized Fab arms. This results in two different LCs and HCs. Enzymatic digestion of mAb2 with trypsin results in 23 peptides each for LC1 and LC2, 52 peptides for HC1 and 39 peptides for HC2. Each mAb2 chain is numbered starting from its N-terminus and labeled with a "T" for the protease trypsin (mAb2 = LC1: 1T - 23T; LC2: 1T - 23T; HC1: 1T - 52T; HC2: 1T - 39T).

### Mass Spectrometer Parameters

For the measurements of the native, reduced and digested antibodies, different settings on the mass spectrometer are used. All mass spectrometer measurements were carried out using an Impact II MS machine by Bruker Daltonics. The parameters for the different measurements are provided below.

| ***MS parameters of the Impact II MS by Bruker Daltonics for the measurement of native antibodies*** | | |
|---|---|---|
| **ESI-Modus** | **Positive** | **Type** |
| Capillary tension | 4500 V | MS |
| End-Plate Offset | 500 V | MS |
| Nebulizer | 2.0 Bar | MS |
| Dry heater Temperature | 180°C | MS |
| Dry gas flow | 3.5 I/min | MS |
| Mass range | 2500 - 4000 m/z | MS/MS |
| Summation | 6029 x | MS/MS |
| Auto MS/MS | Deactivated | MS/MS |
| Fragmentation | Collision-induced dissociation (CID) | MS/MS |

| ***MS parameters of the Impact II MS by Bruker Daltonics for the measurement of reduced antibodies*** | | |
|---|---|---|
| **ESI-Modus** | **Positive** | **Type** |
| Capillary tension | 4500 V | MS |
| End-Plate Offset | 500 V | MS |
| Nebulizer | 2.0 Bar | MS |
| Dry heater Temperature | 220°C | MS |
| Dry gas flow | 11 I/min | MS |
| Mass range | 300 - 3000 m/z | MS/MS |
| Summation | 1,00 Hz | MS/MS |
| Auto MS/MS | Deactivated | MS/MS |
| Fragmentation | Collision-induced dissociation (CID) | MS/MS |

| ***MS parameters of the Impact II MS by Bruker Daltonics for the measurement of peptide mapping*** | | |
|---|---|---|
| **ESI-Modus** | **Positive** | **Type** |
| Capillary tension | 4500 V | MS |
| End-Plate Offset | 500 V | MS |
| Nebulizer | 2.0 Bar | MS |
| Dry heater Temperature | 220°C | MS |
| Dry gas flow | 11 I/min | MS |
| Mass range | 150 - 2000 m/z | MS/MS |
| Summation | 2,00 Hz | MS/MS |
| Auto MS/MS | Activates | MS/MS |
| Fragmentation | Collision-induced dissociation (CID) | MS/MS |

### Experiments

### "Offline" Native Antibody Characterisation

Native antibody characterisation was performed using known manual methods for both mAb1and mAb2 for comparison with the results of the system of the invention.

The samples were separated using a BioZen Intact C4 (2.1 x 150 mm , 3.6 µm) column from Phenomenex on an Acquity UPLC machine from Waters at 20°C using the following parameters:

| **Time [min]** | **Solvent A (0,1% Formic Acid in ddH₂O) in %** | **Solvent B (0,1% Formic Acid in Acetonitrile) in %** | **Flowrate [ml/min]** | **Comments** |
|---|---|---|---|---|
| 0.00 | 80 | 20 | 0.5 | Elution |
| 8.00 | 60 | 40 | 0.5 | |
| 9.00 | 20 | 80 | 0.5 | |
| 10.00 | 20 | 80 | 0.5 | |
| 10.01 | 99 | 1 | 0.5 | Reequilibration of the column |
| 11.00 | 99 | 1 | 0.5 | |
| 11.01 | 20 | 80 | 0.5 | |
| 12.00 | 20 | 80 | 0.5 | |
| 12.01 | 99 | 1 | 0.5 | |
| ... | ... | ... | ... | |
| 14.00 | 80 | 20 | 0.5 | |
| 15.00 | 80 | 20 | 0.5 | |

The sample prepared by this method was analysed by mass spectrometry using the method disclosed above for native antibody measurements. The results are show in Figures 3 to 9 (and discussed in detail below in "Results and Discussion").

### "Offline" Reduced Antibody Characterisation

Reduced antibody characterisation was performed using known manual methods for both mAb1and mAb2 for comparison with the results of the system of the invention.

For sample reduction, 150 µl of denaturation buffer and 10 µl of tris(2-carboxyethyl)phosphine (TCEP) solution were added to 50 µl aliquots of mAb1 and mAb2 (5 mg/ml in MilliQ water) and incubated for 20 minutes at 25° C in a shaker.

Samples were alkylated by addition of 3.2 µl of N-Ethylmaleimide (NEM) solution were and incubated for a further 20 min at 25° C in the shaker. The buffer was changed using NAP-5 columns. The prepared samples were stored at -20°C were.

The reduced mAb1 and mAb2 antibodies were separated using a BioZen Intact C4 (2.1 x 150 mm , 3.6 µm) column from Phenomenex on an Acquity UPLC machine from Waters at 80°C using the following parameters:

| **Time [min]** | **Solvent A (0,1% Formic Acid in ddH₂O) in %** | **Solvent B (0,1% Formic Acid in Acetonitrile) in %** | **Flowrate [ml/min]** | **Comments** |
|---|---|---|---|---|
| 0.00 | 85 | 15 | 0.50 | Loading |
| 2.00 | 85 | 15 | 0.50 | |
| 2.01 | 78 | 22 | 0.50 | Elution |
| 10.00 | 65 | 35 | 0.50 | |
| 11.00 | 55 | 45 | 0.50 | |
| 12.00 | 20 | 80 | 0.50 | |
| 13.00 | 20 | 80 | 0.50 | |
| 14.00 | 99 | 1 | 0.50 | Washing and reequilibration |
| 15.00 | 20 | 80 | 0.50 | |
| 16.00 | 99 | 1 | 0.50 | |
| 17.00 | 20 | 80 | 0.50 | |
| 18.00 | 99 | 1 | 0.50 | |
| 20.00 | 99 | 1 | 0.50 | |

The sample prepared by this method was analysed by mass spectrometry using the method disclosed above for reduced antibody measurements. The results are show in Figures 3 to 9 (and discussed in detail below in "Results and Discussion").

### "Offline" Digested Antibody Characterisation - Peptide Mapping

for both mAb1and mAb2 for comparison with the results of the system of the invention.

Reduced samples were further digested with 20 µl of trypsin solution for 20 hours at 37°C on a shaker. To stop the digestion, 40 µl Trifluoroacetic acid solution was added and samples were then stored at -20°C.

The digested antibody samples were analyzed using an InfinityLab Poroshell 120 SB-C18 (2.1 x 150mm, 1.9µm) pre-column from Agilent Technologies on an Acquity UPLC system at 40°C using the following parameters:

| **Time [min]** | **Solvent A (0,1% Formic Acid in ddH₂O) in %** | **Solvent B (0,1% Formic Acid in Acetonitrile) in %** | **Flowrate [ml/min]** | **Comments** |
|---|---|---|---|---|
| 0.00 | 99 | 1 | 0.4 | Loading |
| 1.00 | 99 | 1 | 0.4 | |
| 7.00 | 85 | 15 | 0.4 | Elution |
| 14.00 | 81 | 19 | 0.4 | |
| 31.00 | 65 | 35 | 0.4 | |
| 35.00 | 30 | 70 | 0.4 | |
| 36.00 | 30 | 70 | 0.4 | |
| 38.00 | 0 | 100 | 0.4 | Washing and reequilibration |
| 39.00 | 99 | 1 | 0.4 | |
| 42.00 | 99 | 1 | 0.4 | |

The sample prepared by this method was analysed by mass spectrometry using the method disclosed above for peptide mapping measurements. The results are show in Figures 3 to 9 (and discussed in detail below in "Results and Discussion").

### "Online" Native Antibody Characterisation

Native antibody characterisation was performed using known LC-MS method for both mAb1and mAb2 for comparison with the results of the system of the invention.

For the separation and fractionation of the native reference standard antibodies mAb1 and mAb2, a TSKgel UP-SW3000 (4.6 x 300 mm, 2.0 µm) column from Tosoh Bioscience was employed as first dimension. Sample was injected into the system and size exclusion chromatography (SEC) was performed at 20°C with a potassium phosphate buffer (0.20mol/I potassium phosphate, 0.25 mol/I potassium chloride, pH 6.2 ± 0.1) at a flow rate of 0.3 ml/min. By detecting the absorbance at 280 nm, the main peaks of mAb1 and mAb2 as well as all LMWs (see Figure 4) of mAb2 were fractionated with the MHC valve and stored in 120 µL loops of deck A and B.

The native antibody fractions were then analyzed using a BioZen Intact C4 (2.1 x 150 mm , 3.6 µm) column from Phenomenex on an Acquity UPLC from Waters at 20°C using the following parameters:

| **Time [min]** | **Solvent A (0,1% Formic Acid in ddH₂O) in %** | **Solvent B (0,1% Formic Acid in Acetonitrile) in %** | **Flowrate [ml/min]** | **Comments** |
|---|---|---|---|---|
| 0.00 | 80 | 20 | 0.50 | Sample loading and salt removal |
| 2.00 | 80 | 20 | 0.50 | |
| 10.00 | 60 | 40 | 0.50 | Elution gradient |
| 19.00 | 20 | 80 | 0.50 | |
| 20.00 | 20 | 80 | 0.50 | |
| 20.01 | 1 | 99 | 0.50 | Washing and reequilibration |
| 21.00 | 1 | 99 | 0.50 | |
| 22.00 | 99 | 1 | 0.50 | |
| 23.00 | 1 | 99 | 0.50 | |
| 24.00 | 99 | 1 | 0.50 | |
| 25.00 | 1 | 99 | 0.50 | |
| 25.01 | 80 | 20 | 0.50 | |
| 30.00 | 80 | 20 | 0.50 | |

The sample prepared by this method was analysed by mass spectrometry using the method disclosed above for native antibody measurements. The results are show in Figures 3 to 9 (and discussed in detail below in "Results and Discussion").

### "Online" Reduced Antibody Characterisation

Reduced antibody characterisation was performed using known LC-MS method for both mAb1and mAb2 for comparison with the results of the system of the invention.

As above, the mAb1 and mAb2 samples were first separated and fractionated using a TSKgel UP-SW3000 (4.6 x 300 mm, 2.0 µm) column from Tosoh Bioscience. Sample was injected into the system and size exclusion chromatography (SEC) was performed at 20°C with a potassium phosphate buffer (0.20mol/I potassium phosphate, 0.25 mol/I potassium chloride, pH 6.2 ± 0.1) at a flow rate of 0.3 ml/min. By detecting the absorbance at 280 nm, the main peaks of mAb1 and mAb2 as well as all LMWs (see Figure 4) of mAb2 were fractionated with the MHC valve and stored in 120 µL loops of deck A and B.

The fractions were then reduced and subsequently analyzed using a BioZen Intact C4 (2.1 x 150 mm , 2.6 µm) column from Phenomenex on an Acquity UPLC from Waters at 80°C using the following parameters:

| **Time [min]** | **Solvent A (0,1% Formic Acid in ddH₂O) in %** | **Solvent B (0,1% Formic Acid in Acetonitrile) in %** | **Solvent C (50mM Tris(2-Carboxyethyl)phosphine in ddH2O) in %** | **Flowrate [ml/min]** | **Comments** |
|---|---|---|---|---|---|
| 0.00 | 0 | 0 | 100 | 0.50 | Sample loading and reduction |
| 5.00 | 0 | 0 | 100 | 0.50 | |
| 10.00 | 99 | 1 | 0 | 0.50 | |
| 11.00 | 78 | 22 | 0 | 0.50 | Elution gradient |
| 19.00 | 65 | 35 | 0 | 0.50 | |
| 20.00 | 55 | 45 | 0 | 0.50 | |
| 21.00 | 20 | 80 | 0 | 0.50 | |
| 22.00 | 99 | 1 | 0 | 0.50 | Washing and reequilibration |
| 23.00 | 20 | 80 | 0 | 0.50 | |
| 24.00 | 99 | 1 | 0 | 0.50 | |
| 25.00 | 20 | 80 | 0 | 0.50 | |
| 26.00 | 99 | 1 | 0 | 0.50 | |
| 28.00 | 99 | 1 | 0 | 0.50 | |

The sample prepared by this method was analysed by mass spectrometry using the method disclosed above for reduced antibody measurements. The results are show in Figures 3 to 9 (and discussed in detail below in "Results and Discussion").

### "Online" Digested Antibody Characterisation - Peptide Mapping

Peptide mapping using the known multidimensional LC-MS system of Oezipek et al. (discussed in the background section) was carried out for both mAb1and mAb2 for comparison with the results of the system of the invention.

### ¹D Fractionation Module - Ion Exchange Chromatography and Fractionation

The 1D method varies according on the product being analyzed and corresponds to the GxP-method for IEC or SEC quality control (QC) analysis. For the characterization of Herceptin^{®} (Trastuzumab) charge variants, a MAbPac^{™} WCX (4.0 x 250 mm, 10.0 µm) column from Thermo Fisher Scientific^{™} was employed as first dimension. Unstressed Herceptin^{®} (150 µg) was injected into the system and the parameters of supplementary table S3 were chosen for the cation exchange chromatography (CEX). By detecting the absorbance at 214 nm the acidic, main and basic peaks where fractionated with the MHC valve and stored in 120 µL loops of deck A and B. For reduction (2D), digestion (3D), trapping (4D) and peptide mapping analysis (5D) the fractions where subsequently processed with the following dimensions.

Ion exchange chromatography was used in the fractionation module in the system of the examples. Product specific 'D CEX parameters and gradients are listed for Herceptin (trastuzumab) and the bi-specific mAb (BsMAb). For the Herceptin CEX a Thermo Scientific ProPac" WCX-10 Analytical, 4 x 250mm column was used at 25°C. The absorbance was measured with the VWR detector at 214 nm. The BsMAb CEX was performed with a YMC BioPro IEX-SF, 100 x 4.6 mm, 5 p.m column at 40°C and the absorbance was measured at 280 nm.

The following parameters and timings were used in the fractionation:

| **Fractionation Module set up- ¹D-Pump Cation Exchange Chromatography Herceptin (trastuzumab)** | | | | | |
|---|---|---|---|---|---|
| **Time [min]** | **Flowrate [mL/min]** | **Eluent A [%] (10 inIVI sodium phosphate, pH 7.5)** | **Eluent B [%] *(*10 m/V1 sodium phosphate, 100 inIVI NaC1 pH 7.5)** | | **Comment** |
| 0.00 | 0.8 | | 15 | | CEX and peak fractionation by multiple heart cutting |
| 30.00 | | | 55 | | |
| 35.00 | | | 55 | | |
| 36.00 | | | 10 | | Wash to reduce carryover |
| | | | 0 | | |
| 44.00 | | | 10 | | |
| | | | 0 | | |
| 45.00 | | | 15 | | Column equilibration |
| 55.00 | | | 15 | | |

| **Fractionation Module Set Up - 'D-Pump Cation Exchange Chromatography BsMAb** | | | | | |
|---|---|---|---|---|---|
| **Time [min]** | **Flowrate [mL/min]** | **Eluent A [%] (20 mM BES, pH 6.8)** | **Eluent B [%] (20 in/14 BES, 500 inIYI NaCt pH 6.8)** | | **Comment** |
| 0.00 | 0.8 | | 2 | | CEX and peak fractionation by multiple heart cutting |
| 5.00 | | | 2 | | |
| 35.00 | | | 15 | | |
| 35.10 | | | 10 | | Wash to reduce carryover |
| | | | 0 | | |
| 40.00 | | | 10 | | |
| | | | 0 | | |
| 40.10 | | | 2 | | Column equilibration |
| 50.00 | | | 2 | | |
| | | | | | S5 |

| | | | | | |
|---|---|---|---|---|---|
| ²D Reduction Module - On-Column Reduction | | | | | |

The second dimension of the mD-UHPLC-MS/MS instrument incorporates a Poroshell 300SB-C3 (2.1 x 12.5 mm, 5.0 µm) cartridge from Agilent Technologies for trapping and reduction of the 1D fractions. The fast on-column reduction was performed by flushing the trapped mAbs with 20 mM Tris-(2-carboxyethyl)-phosphin (TCEP). Afterwards, the C3-Cartridge was washed and the reduced mAbs were eluted onto the immobilized enzyme reactor (IMER).

The reduction was performed on a Poroshell 3005B-C3 2.1 x 12.5 mm, S.0 um (Agilent Technologies) cartridge at 40°C. The following parameters and timings were used in the reduction:

| **Reduction - ²D-Pump On-Column Reduction** | | | | | | | |
|---|---|---|---|---|---|---|---|
| **Time [min]** | **Flow rate [mL/ min]** | **Fluent AI [%] (20 mNITCEP, 5% ACN in H2O)** | **Fluent B1 [%] (0.1% FA, 5% ACN in H2O)** | **Fluent A2 [%] (0.1% FA in H2O)** | **Fluent B2 [%] (0.1% FA in ACN)** | **Comment** | |
| 0.00 | 0.50 | 0 | 100 | | | Start instrument 2 (²D/³D/⁴D) | |
| 0.01 | | 100 | 0 | | | ²D column trapping and on-column reduction | |
| 4.00 | | 100 | 0 | | | | |
| 4.01 | 2.00 | | | 85 | 15 | Solvent selection valve switch | |
| 7.00 | | | | 85 | 15 | AI/ B1 4 A2/ B2 and wash | |
| 7.01 | 0.50 | | | 50 | 50 | Valve 1 switch 4 | 4 |
| 7.25 | | | | 50 | 50 | Elution of reduced mAbs | |
| 8.00 | | | | 50 | 50 | | |
| 8.01 | | | | 100 | 0 | | |
| 8.50 | | | | 100 | 0 | | |
| 8.51 | | | | 50 | 50 | | |
| 11.50 | | | | 50 | 50 | | |
| 11.51 | | | | 100 | 0 | | |
| 12.00 | | | | 100 | 0 | | |
| 12.01 | | | | 50 | 50 | | |
| 15.00 | | | | 50 | 50 | | |
| 15.01 | | | | 100 | 0 | | |
| 15.50 | | | | 100 | 0 | | |
| 15.51 | | | | 50 | 50 | | |
| 18.50 | | | | 50 | 50 | | |
| 18.51 | | | | 100 | 0 | | |
| 19.00 | | | | 100 | 0 | | |
| 19.01 | | | | 50 | 50 | | |
| 22.00 | | | | 50 | 50 | | |
| 22.01 | | | | 50 | 50 | Valve 1 switch waste | 4 |
| 23.00 | 1' 00 | | | 5 | 95 | Wash to reduce carryover | |
| 23.01 | | | | 95 | 5 | | |
| 24.00 | | | | 5 | 95 | | |
| 24.01 | | | | 95 | 5 | | |
| 25.00 | | | | 5 | 95 | | |
| 25.01 | | | | 95 | 5 | | |
| 26.00 | | | | 5 | 95 | | |
| 26.01 | | | | 95 | 5 | | |
| 27.00 | | | | 5 | 95 | | |
| 27.01 | | | | 95 | 5 | | |
| 28.00 | | | | 5 | 95 | | |
| 28.01 | | | | 95 | 5 | | |
| 29.00 | | | | 5 | 95 | | |
| 29.01 | | | | 95 | 5 | | |
| 30.00 | | | | 5 | 95 | | |
| 35.00 | | | | 5 | 95 | | |
| 35.01 | 0.5 | 50 | 50 | | | Solvent selection valve switch | |
| 40.00 | | 50 | 50 | | | A2/ B2 4 Ai/ si | |
| 40.01 | | 0 | 100 | | | Column equilibration | |
| 60.00 | | 0 | 100 | | | | |

| | | | | | | | |
|---|---|---|---|---|---|---|---|
| ³D Digestion Module - On-Column Digestion | | | | | | | |

For online digestion of the reduced 1D fractions, a custom made LysC (2.1 x 100 mm, Perfinity Biosciences) and/ or Trypsin (2.1 x 100 mm, Perfinity Biosciences) IMER was used as third dimension. Thus, either an in-parallel or single enzymatic digestion setup can be selected with the mD-UHPLC-MS/MS instrument. For the parallel setup the flow is split in half in front of the columns and merged afterwards by two T-pieces. This allows a separated digestion with both columns and afterwards the combined analysis of LysC and Trypsin peptides. In addition, the mD-UHPLC-MS/MS system allows a single enzymatic digestion setup where only one column is installed and the remaining ports of the T-pieces are blocked by stop plugs. For optimal digestion, the reduced 1D fractions are diluted with digestion buffer at a ratio of 1: 6 with a biocompatible 100 µL binary mixer from ASI-Analytical Scientific Instruments. During the digestion step the IMER was connected in-line with the peptide trapping column and the flow-through digestion took approximately 70 seconds.

For the online digestion a custom made LysC (2.1 x 100 mm, Perfinity Biosciences) and/ or a trypsin (2.1 x 100 mm, Perfinity Biosciences) immobilized enzyme reactor (IMER) was used at 40°C.

The following parameters and timings were used in the digestion:

| **³D-Pump On-Column Digestion** | | | | | | | |
|---|---|---|---|---|---|---|---|
| **Time [min]** | **Flowrate [mL/min]** | **Eluent A [%] (SO mM MS, 10 m/VI Ca02, pH 8.5)** | | **Eluent B [%] (ACN)** | | **Comment** | |
| 0.00 | 0.25 | 100 | | 0 | | Start nstrument 2 (²D/³D/⁴D) | |
| 0.01 | 1.50 | 50 | | 50 | | Wash to reduce carryover | |
| 4.00 | | 50 | | 50 | | | |
| 4.01 | | 100 | | 0 | | | |
| 6.00 | | 100 | | 0 | | | |
| 6.01 | 0.25 | 100 | | 0 | | | |
| 7.01 | | 100 | | 0 | | Valve 1 switch 4 | 4 |
| 7.02 | | 100 | | 0 | | Digestion of reduced mAbs | |
| 22.00 | | 100 | | 0 | | | |
| 22.01 | | 100 | | 0 | | Valve 1 switch 4 | 4 |
| 25.00 | | 100 | | 0 | | Wash to reduce carryover | |
| 25.01 | 1 50 | 50 | | 50 | | | |
| 30.00 | | SO | | 50 | | | |
| 30.01 | | 100 | | 0 | | | |
| 31.00 | | 100 | | 0 | | | |
| 31.01 | | 50 | | 50 | | | |
| 32.00 | | 50 | | 50 | | | |
| 32.01 | | 100 | | 0 | | | |
| 33.00 | | 100 | | 0 | | | |
| 33.01 | | 50 | | 50 | | | |
| 34.00 | | 50 | | 50 | | | |
| 34.01 | | 100 | | 0 | | | |
| 35.00 | 0.25 | 100 | | 0 | | Column equilibration | |

| | | | | | | | |
|---|---|---|---|---|---|---|---|
| ⁴D Trapping Module - Pre-Column Trapping | | | | | | | |

After digestion the eluting peptides were diluted with Milli-Q H2O at a ratio of 1: 5.5 using a biocompatible 150 µL binary mixer from ASI- Analytical Scientific Instruments. The two dilution steps (3D, 4D) result in a final acetonitrile concentration of min. 1% for peptide trapping depending on the used pre column. For the Herceptin^{®} analysis an InfinityLab Poroshell 120 SB-C18 (3.0x 5 mm, 1.9 µm) pre-column from Agilent Technologies was used. For the bispecific mAb (BsMAb) analysis an ACQUITY UHPLC BEH C18 VanGuard precolumn (2.1 x 5 mm, 1.7 µm) from Waters Corporation was incorporated into the system. After peptide trapping the pre-column was washed and subsequently placed in-line with the analytical full length UHPLC-column for peptide mapping analysis.

In the examples, peptide trapping was performed on a trapping (pre)column, which matched with the main sepaeration column. For Herceptin an InfinityLab Poroshell 120 SB-C18 3.0x S mm, 1.9 vm (Agilent Technologies) precolumn was used at 30°C. For the BsMAb analysis an ACQUITY UHPLC BEH C18 2.1x Smm, 1.7 p.m (Waters Corporation) precolumn was used at 30°C and switched to 45°C one minute prior peptide mapping analysis (24 min.

The following parameters and timings were used in the trapping:

| **Trapping (⁴D-)Pump Peptide Trapping** | | | | | | | | | |
|---|---|---|---|---|---|---|---|---|---|
| **Time [min]** | **Flowrate [mL/min]** | **Eluent A [%] (0.1%FA in H2O)** | **Eluent B [%] (0.1% FA in ACN)** | **Eluent C [%] (H2O)** | **Eluent D [%] (ACN)** | **Comment** | | | |
| 0.00 | 0.25 | 95 | 5 | 0 | 0 | Start instrument 2 (²D/³D/⁴D) | | | |
| 0.01 | 1.00 | 95 | 5 | 0 | 0 | Wash to reduce carryover | | | |
| 1.00 | | 5 | 95 | 0 | 0 | | | | |
| 2.00 | | 5 | 95 | 0 | 0 | | | | |
| 2.01 | | 95 | 5 | 0 | 0 | | | | |
| 3.00 | | 5 | 95 | 0 | 0 | | | | |
| 4.00 | | 5 | 9S | 0 | 0 | | | | |
| 4.01 | | 99 | 1 | 0 | 0 | | | | |
| 5.00 | | 0 | 0 | 100 | 0 | | | | |
| 7.01 | 1.35 - 2.20 (depending on precolu mn) | 0 | 0 | 100 | 0 | Valve 1 | 4 | 4 | |
| 7.02 | | 0 | 0 | 100 | 0 | ⁴D column trapping | | | |
| 22.00 | | 0 | 0 | 100 | 0 | | | | |
| 22.01 | | 0 | 0 | 99 | 1 | Valve 1 switch ⁴D 4 waste | | | |
| 22.02 | | 0 | 0 | 99 | 1 | Wash to reduce salt concentration | | | |
| 25.00 | | 0 | 0 | 99 | 1 | | | | |
| 25.01 | 0.10 | 95 | 5 | 0 | 0 | Valve 2 switch ⁵D 4 MS | | | 4 |
| 59.00 | | 95 | 5 | 0 | 0 | Valve 2 switch waste | | | 4 |
| 59.01 | 0.25 | 95 | 5 | 0 | 0 | Column equilibration | | | |

| | | | | | | | | | |
|---|---|---|---|---|---|---|---|---|---|
| ⁵D Separation Module - Peptide Mapping Analysis | | | | | | | | | |

The peptide mapping analysis was initiated by switching the pre-column in-line with the analytical reversed phase column. The used analytical column depends on the antibody to be investigated. For the Herceptin^{®} analysis an InfinityLab Poroshell 120 SB-C18 (2.1x 150 mm, 1.9 µm) column from Agilent Technologies was used. The chromatographic peptide separation for the BsMAb is performed by using an UHPLC BEH Peptide C18 column (2.1 mm x 150mm, 1.7 µm) from Waters Corporation. The parameters and gradients are given in supplementary table S7. For detection of MS1 and MS2 spectra, the high-resolution ESI-Q-ToF mass spectrometer Impact II from Bruker Daltonics was used.

The peptide mapping analysis was performed on an UHPLC-column, depending on the analyzed mAb. For Herceptin an InfinityLab Poroshell 120 SB-C18 2.1 x 150 mm, 1.9 vm (Agilent Technologies) UHPLC-column was used at 40°C. For the BsMAb analysis an Waters ACQUITY UPLC Peptide BEH C18 Column, 300A, 1.7 vm, 2.1 mm X 150 mm (Waters Corporation) UPLC-column was used at 40°C. For both columns a flowrate of 0.4 mL/min was used.

The following parameters and timings were used in the peptide mapping:

| **Separation (⁵D-)Pump Peptide Mapping Analysis** | | | | | | |
|---|---|---|---|---|---|---|
| **Time [min]** | **Fluent A [%] (0.1% FA in H2O)** | | **Fluent B [%] (0.1% FA in ACN)** | | **Comment** | |
| 0.00 | 95 | | 5 | | Start instrument 2 (²D/³D/⁴D) | |
| 0.01 | 95 | | 5 | | Wash to reduce carryover | |
| 2.00 | 5 | | 95 | | | |
| 3.00 | 5 | | 95 | | | |
| 3.01 | 95 | | 5 | | | |
| 5.00 | 5 | | 95 | | | |
| 6.00 | 5 | | 95 | | | |
| 6.01 | 95 | | 5 | | | |
| 8.00 | 5 | | 95 | | | |
| 9.00 | 5 | | 95 | | | |
| 9.01 | 95 | | 5 | | | |
| 11.00 | 5 | | 95 | | | |
| 12.00 | 5 | | 95 | | | |
| 15.00 | 99 | | 1 | | | |
| 25.01 | 99 | | 1 | | Valve 2 switch | 4 'D 4 MS |
| 65.00 | 60 | | 40 | | Peptide mapping analysis | |
| 68.00 | 40 | | 60 | | | |
| 69.00 | 5 | | 95 | | | |
| 70.00 | 5 | | 95 | | | |
| 70.01 | 95 | | 5 | | Wash to reduce carryover | |
| 71.00 | 5 | | 95 | | | |
| 72.00 | 5 | | 95 | | | |
| 72.01 | 95 | | 5 | | | |
| 73.00 | 5 | | 95 | | | |
| 74.00 | 5 | | 95 | | | |
| 74.01 | 95 | | 5 | | Valve 2 switch waste | 4 waste ⁵D 4 |
| 75.00 | | 95 | | 5 | Column equilibration | |

The sample prepared by this method was analysed by mass spectrometry using the method disclosed above for peptide mapping measurements. The results are show in Figures 3 to 9 (and discussed in detail below in "Results and Discussion").

### Instrument set Up

The mD-UHPLC-MS/MS system used in the examples is configured as two instruments within the Open Lab software package (ChemStation). The communication between the two instruments was performed by a custom made macro "valve event plugin" from ANGI (Gesellschaft fur angewandte Informatik, Karlsruhe, Germany).

| **Instrument 1 ('D/⁵D)** | | |
|---|---|---|
| **Module** | **Product** | **Product No.** |
| Autosampler | 1260 HiP Bio ALS | G5667A |
| Fractionation ('D-)Pump | 1290 binary pump | G4220A |
| 'D-Pump | 1290 binary pump | G4220A |
| Multiple Heart Cutting Valve (MHC) | 2-position/4-port duo-valve | G4236A (MHC-kit) |
| | | 5067-4214 (valve) |
| Loop Deck A with 6 sampling loops (120 id, Volume) | 6-position/14-port valve | G4242A (MHC upgrade kit) |
| | | 5067-4142 (valve) |
| Loop Deck B with 6 sampling loops (120 itL Volume) | 6-position/14-port valve | G4242A |
| | | 5067-4142 (valve) |
| UV detector 1 | 1260 VWD | G1314F |

| **Instrument 2 (²D/³D/⁴D)** | | |
|---|---|---|
| **Module** | **Product** | **Product No.** |
| Reduction (² D-)Pump | 1290 binary pump | G4220A |
| Digestion (³D-)Pump | 1260 quaternary pump | G1311B |
| Trapping (⁴D-)Pump | 1260 quaternary pump | G1311B |
| Column compartment 1 (CC1) installed 2-position/10-port valve (800 bar) | 1290 TCC | G1316C (CC1) |
| | | 5067-4283 (valve) |
| Column compartment 2 (CC2) installed 2-position/10-port valve (1300 bar) | 1290 MCT | G1314F (CC2) |
| | | 5067-4240 (valve) |

### 3-in-1 mD-UHPLC-MS/MS

The system of the invention used in these examples is a multidimensional LC System coupled to a mass spectrometer and operating a UHPLC separating column. The system (sometimes referred to as "3-in-1 mD-UHPLC-MS/MS") is based on LC-modules from Agilent Technologies (Waldbronn, Germany) coupled with the high-resolution mass spectrometer Impact II from Bruker Daltonics. The system of the invention used in the examples is shown schematically in Figure 1A and in more detail in Figure 2.

Reagents for the analysis with the 3-in-1 mD-UHPLC-MS/MS instrument are listed above under 'Reagents".

The system is configured as two instruments within the OpenLab software package from Agilent Technologies. For communication between the two instruments a self-designed macro "valve event plugin" from ANGI (Gesellschaft für angewandte Informatik, Karlsruhe, Germany) was used. The macro starts the method of the second instrument and the mass spectrometer via a contact closure signal for each fraction in the first dimension.

An overview of each "dimension" (or module) in the system is provided directly below. Following the overview, details of the instrument set up and operating parameters used in each module in each of the examples is also provided. A schematic of the instrument set-up used in the examples is shown in Figures 1A and in more detail in Figure 2.

The samples prepared by this method were analysed by mass spectrometry using the method disclosed above for native antibody measurements, reduced antibody measurements and peptide mapping analysis. The results are show in Figures 3 to 9 (and discussed in detail below in "Results and Discussion").

### ¹D - Fractionation Module - Ion Exchange Chromatography and Fractionation

The 1D method corresponds to the GxP-method (e.g. IEC, SEC. HIC or ProteinA) for quality control (QC) analysis For the characterization of mAb1 and mAb2, a TSKgel UP-SW3000 (4.6 x 300 mm, 2.0 µm) column from Tosoh Bioscience was employed as first dimension. Sample was injected into the system and size exclusion chromatography (SEC) was performed at 20°C with a potassium phosphate buffer (0.20mol/I potassium phosphate, 0.25 mol/I potassium chloride, pH 6.2 ± 0.1) at a flow rate of 0.3 ml/min.

The table below lists the entire method:

| **Time [min]** | **Solvent A [%]** (0.20mol/I potassium phosphate, 0.25 mol/I potassium chloride, pH 6.2 ± 0.1) | **Flowrate [ml/min]** | **Comment** |
|---|---|---|---|
| 0.0 | 100 | 0.3 | Separation |
| 25.0 | 100 | 0.3 | |
| 25.1 | 100 | 0.3 | Solvent saving & wash |
| 60.0 | 100 | 0.1 | |
| 70.0 | 100 | 0.3 | Reequilibration |
| 82.0 | 100 | 0.3 | |

By detecting the absorbance at 280 nm, the main peaks of mAb1 and mAb2 as well as all LMWs (see Figure 4) of mAb2 were fractionated with the MHC valve and stored in 120 µL loops of deck A and B.

For de-salting (²D) when present, reduction (³D), digestion (⁴D), trapping (⁵D) and peptide mapping (⁶D) the fractions were subsequently processed with the following dimensions.

After fractionation the stored samples were pumped out of the MHC sequentially and analysed in turn.

First each sample was passed through a first splitter with a flow rate of 0.5 ml/min. Some of the sample was pumped to the ²D buffer exchange module as the native antibody sample to be analysed and the rest of the sample was pumped to the ³D reduction module.

### ²D - Buffer Exchange Module

In the buffer exchange module a BioZen Intact C4 with the masses 2.1 × 150 mm and a pore size of 3.6 µm from Phenomenex is used.

The column is equilibrated at 20°C, a flow of 0.5 ml/min and an eluent ratio of 75% eluent A 0.1% FA ddH₂O and 25% eluent B 0.1% FA ACN.

For the transfer of the sample from the first dimension, the flow is stopped in order not to create back pressure for the grit. After the transfer and switching of the switching valves 3 and 4 to close the splitters, the salts of the fractionation buffer are washed off the C4 column for two minutes. For this, a flow of 0.5 ml/min and 25% B is used.

After the wash, the native measurement by mass spectrometer starts. The gradient rises to 40% B in the first six minutes. The gradient then rises to 80% B within one minute and remains at 80% B for another one and a half minutes ().

The table below lists the entire method in terms of gradient and parameters of the buffer exchange module of the 3 in 1 method

| **Time [min]** | **Solvent A [%]** (200 mM Ammoniumacetate pH 7.0) | **Flowrate [ml/min]** | **Comment** |
|---|---|---|---|
| 0.0 | 100 | 0.3 | |
| 10.0 | 100 | 0.3 | Buffer Exchange and Native Separation |
| 10.1 | 100 | 0.1 | Solvent saving & wash |
| 60.0 | 100 | 0.1 | |
| 70.0 | 100 | 0.3 | Reequilibration |
| 82.0 | 100 | 0.3 | |

From the size exclusion column, the native antibody sample and the reduced antibody sample are sent sequentially to the mass spectrometer for analysis.

### ³D - Reduction Module - On-Column Reduction

The third dimension of the mD-UHPLC-MS/MS instrument incorporates a PLRP-S 1000A (5µm, 2.1x50mm) column from Agilent for reduction of the 1D fractions. The fast on-column reduction was performed using the following parameter:

| **Time [min]** | **Solvent A [%]** (0,1% Formic Acid in ddH₂O) | **Solvent B [%]** (0,1% Formic Acid in Acetonitrile) | **Solvent C [%]** (50mM DTT in ddH2O) | **Flowrate [ml/min]** | **Comments** |
|---|---|---|---|---|---|
| 0.0 | 95 | 5 | 0 | 0.5 | Loading column |
| 10.0 | 95 | 5 | 0 | 0.5 | |
| 10.1 | 0 | 0 | 100 | 0.5 | Reduction |
| 15.0 | 0 | 0 | 100 | 0.5 | |
| 15.1 | 90 | 10 | 0 | 1.0 | Washing column |
| 20.0 | 90 | 10 | 0 | 1.0 | |
| 20.1 | 90 | 20 | 0 | 0.3 | Elution |
| 30.0 | 30 | 70 | 0 | 0.3 | |
| 30.1 | 5 | 95 | 0 | 0.5 | Washing and reequilibration |
| 31.0 | 5 | 95 | 0 | 0.5 | |
| 31.1 | 95 | 5 | 0 | 0.5 | |
| 32.0* | 5* | 95* | 0* | 0.5* | |
| 32.1* | 95* | 5* | 0* | 0.5* | |
| *repetition of washing steps 10x | | | | | |
| 80.0 | 95 | 5 | 0 | 0.5 | |
| 82.0 | 95 | 5 | 0 | 0.5 | |

Afterwards, the reduced mAbs were eluted using a splitter onto either the immobilized enzyme reactor (IMER) for 4D, or the C4 column for 7D.

### ⁴D Digestion Module - On-Column Digestion

For online digestion of the reduced fractions, a Trypsin (2.1 x 100 mm, Perfinity Biosciences) IMER column at 40°C was used as third dimension.

The column was activated with a flow of 0.25 ml/min, 5% eluent B ACN and 95% eluent A 50 mM Tris and 10 mM CaCl2 at pH 8.5. At the beginning of the analysis, 100% eluent A is used. As soon as the reduced antibodies from the second dimension are eluted via the third dimension, the gradient of the 2 D mixes with the digestion buffer of the 3D. After digestion, the trypsin column is cleaned with 5% B and 95% eluent A for five minutes and reactivated.

| **Time [min]** | **Solvent A (50mM Tris,** 10mM CaCl₂, **pH 8,5) in %** | **Solvent B ((0,1% Formic Acid in Acetonitrile) in %** | **Flowrate [ml/min]** | **Comments** |
|---|---|---|---|---|
| 0.0 | 100 | 0 | 0.25 | Digestion |
| 30.0 | 100 | 0 | 0.25 | |
| 30.1 | 50 | 50 | 0.25 | Washing |
| 50.0 | 50 | 50 | 0.25 | |
| 50.1 | 100 | 0 | 0.25 | Reequilibration |
| 82.0 | 100 | 0 | 0.25 | |

### ⁵D Trapping Module - Pre-Column Trapping

For peptide trapping, an InfinityLab Poroshell 120 SB-C18 (3.0x 5 mm, 1.9 µm) pre-column from Agilent Technologies , and a T-piece were used. The T-piece has the function of reducing the acetonitrile content of the gradient from the second dimension to allow trapping of even smaller peptides with the fourth dimension. The peptide trapping was carried out at 30°C using the parameters below. Briefly, the column was washed for seven minutes before increasing the flow to 1.35 ml/min. At minute ten, 100% ddH2O was used to reduce the acetonitrile content from the 2D. After 20 minutes, the 2D and 3D were taken out of the row with the 4D, then the trapping column was rinsed to remove the salts from the digestion buffer. Finally, the flow was reduced to 0.1 ml/min.

| **Time [min]** | **Solvent A (0,1% Formic Acid in ddH₂O) in %** | **Solvent B (0,1% Formic Acid in Acetonitrile) in %** | **Solvent C (ddH₂O) in %** | **Solvent D (Acetonitrile) in %** | **Flowrate [ml/min]** | **Comments** |
|---|---|---|---|---|---|---|
| 0.00 | 1 | 99 | 0 | 0 | 0.50 | Washing column |
| 7.00 | 1 | 99 | 0 | 0 | 0.50 | |
| 7.01 | 0 | 0 | 99 | 1 | 0.50 | Column Equilibration, Elution and Washing |
| 10.00 | 0 | 0 | 100 | 0 | 1.35 | |
| 32.00 | 0 | 0 | 100 | 0 | 1.35 | |
| 32.01 | 0 | 0 | 100 | 0 | 0.10 | Flow reduction |

### ⁶D Separation Module - Peptide Mapping Analysis

The separation module includes an InfinityLab Poroshell 120 SB-C18 2.1 × 150 mm with a pore size of 1.9 µm. It is equilibrated at 40 ° C with 99% eluent A 0.1% FA in ddH₂O and 1% eluent B 0.1% FA in ACN and a flow of 0.4 ml / min.

The gradient and parameters of the peptide mapping of the 3 in 1 method are outlined in the table below.

| **Time [min]** | **Solvent A (0,1% Formic Acid in ddH₂O) in %** | **Solvent B (0,1% Formic Acid in Acetonitrile) in %** | **Flowrate [ml/min]** | **Comments** |
|---|---|---|---|---|
| 0.00 | 1 | 99 | 0.40 | Column washing and equilibration |
| 1.00 | 99 | 1 | 0.40 | |
| 2.00 | 1 | 99 | 0.40 | |
| ... | ... | ... | ... | |
| 27.00 | 1 | 99 | 0.40 | |
| 27.01 | 99 | 1 | 0.40 | |
| 32.00 | 99 | 1 | 0.40 | |
| 32.01 | 99 | 1 | 0.40 | Elution |
| 72.00 | 60 | 40 | 0.40 | |
| 77.00 | 40 | 60 | 0.40 | |
| 78.00 | 20 | 80 | 0.40 | |
| 79.00 | 80 | 20 | 0.40 | Washing |
| 80.00 | 20 | 80 | 0.40 | |
| 81.00 | 80 | 20 | 0.40 | |
| 82.00 | 20 | 80 | 0.40 | |

### Valve Timetable

The valve timetable below shows the switching time points for each valve except for the MHC or fractionation valve. The MHC or fractionation valve is switched individually for each 1D chromatographic separation and the corresponding retention times of the peaks of interest

| **Time [min]** | **Valve 1 (V1) Position** | **Valve 2 (V2) Position** | **Valve 3 (V3) Position** | **Valve 4 (V4) Position** | **Comment** |
|---|---|---|---|---|---|
| 0.0 | 1 →2 | 1 →10 | 1 →10 | 1 →6 | Start |
| 0.1 | 1 →10 | 1 →10 | 1 →10 | 1 →6 | Desalting native SEC |
| 10.1 | 1 →2 | 1 →10 | 1 →10 | 1 →6 | Reduction & wash |
| 20.1 | 1 →2 | 1 →2 | 1 →10 | 1 →2 | Elution reduced mAbs |
| 30.1 | 1 →2 | 1 →10 | 1 →10 | 1 →6 | preC18 wash |
| 32.1 | 1 →2 | 1 →10 | 1 →2 | 1 →2 | C18 Peptide Mapping |
| 80.0 | 1 →2 | 1 →10 | 1 →10 | 1 →6 | Starting condition |

### Instrument set Up

The mD-UHPLC-MS/MS system of the invention used in the examples is configured as two instruments within the OpenLab software package (ChemStation). The communication between the two instruments was performed by a custom made macro "valve event plugin" from ANGI (Gesellschaft fur angewandte Informatik, Karlsruhe, Germany).

| **Instrument 1 ('D/⁵D)** | | |
|---|---|---|
| **Module** | **Product** | **Product No.** |
| Autosampler | 1260 HiP Bio ALS | G5667A |
| Fractionation ('D-)Pump | 1290 binary pump | G4220A |
| 'D-Pump | 1290 binary pump | G4220A |
| Multiple Heart Cutting Valve (MHC) | 2-position/4-port duo-valve | G4236A (MHC-kit) |
| | | 5067-4214 (valve) |
| Loop Deck A with 6 sampling loops (120 id, Volume) | 6-position/14-port valve | G4242A (MHC upgrade kit) |
| | | 5067-4142 (valve) |
| Loop Deck B with 6 sampling loops (120 itL Volume) | 6-position/14-port valve | G4242A |
| | | 5067-4142 (valve) |
| UV detector 1 | 1260 VWD | G1314F |

| **Instrument 2 (²D/³D/⁴D)** | | |
|---|---|---|
| **Module** | **Product** | **Product No** |
| Desalting (⁰D-)Pump | 1290 binary pump | G4220A |
| Reduction (²D-)Pump | 1290 binary pump | G4220A |
| Digestion (³D-)Pump | 1260 quaternary pump | G1311B |
| Trapping (⁴D-)Pump | 1260 quaternary pump | G1311B |
| Column compartment 1 (CC1) installed 2-position/10-port valve (800 bar) | 1290 TCC | G1316C (CC1) |
| | | 5067-4283 (valve) |
| Column compartment 2 (CC2) installed 2-position/10-port valve (1300 bar) | 1290 MCT | G1314F (CC2) |
| | | 5067-4240 (valve) |

### Results and Discussion

In the following chapter, the results of the "offline", "online" and 3-in-1 mD UHPLC MS/MS analysis of mAb1 and mAb2 are provided. The term 3-in-1 mD UHPLC MS/MS refers to the system of the invention and is also shorted to "3-in-1 method".

The evaluation of the results for the peptide mapping was carried out with the Software Byos from Protein Metrics.

The evaluation of the native and reduced results of the mAb1 and mAb2 was carried out using DataAnalysis from Bruker.

Alkylated samples were used for the offline measurements to prevent the formation of new disulfide bridges. This leads to a mass shift of 125,125 Daltons per alkylation site in reduced measurement and offline peptide mapping.

To provide all 3 sets of data (native, reduced and peptide mapping) the offline methods require at least 24 hours. 24 hours is achievable only if the native measurement is running during the sample preparation of the reduced mAbs and the reduction measurement during the sample preparation of the digested mAbs. 24 hours here does not take into account the time needed to prepare buffers for which half a day would also have to be calculated.

To provide all 3 sets of data (native, reduced and peptide mapping) using the individual online methods take a total of 170 minutes (2h 50 minutes). The 170 minutes does not take into account the time required for converted a single LC/MC system to run each method inf sequence. An alternative for individual online measurements is to use several LC/MS systems. This is of course associated with a significant additional cost in the purchase.

The 3-in-1 method of the invention takes 87 minutes to provide all 3 sets of data (native, reduced and peptide mapping). This is only 5 minutes longer than the online peptide mapping method.

The 3-in-1 method of the invention provides the same results as the offline and the individual online methods. Furthermore, the susceptibility to errors by the analyst is minimized, since in the 3-in-1 method only the eluents and the dilution are carried out by the analyst.

Compared to offline analytics, the 3-in-1 method has the advantage that artifacts caused by the offline process are completely omitted.

In addition, the robustness of the measurements is maintained, as one and the same fraction is used for native measurement, reduced measurement and peptide mapping, while offline and individual online analysis involves measurements with different fractions from several injections.

### Native Measurements of mAb 1

The native measurements of the mAb1 are preferent in Figure 3 which contains the total ion chromatograms (TIC) of the measurements. Figure 3A shows the offline measurement, figure 3B shows the online measurement and Figure 3C shows the measurements from the 3-in-1 mD UHPLC MS/MS of the invention.

The offline chromatogram (Figure 3 A) has a peak at minute 9 with an intensity of 5 x 10⁶. In the online chromatogram of the native measurement, Figure 3B, there is also a peak at minute 9 with an intensity of about 1.25 x 10⁷. In the 3 in 1 measurement, Figure 3C, a peak can be seen at minute 7.9 with an intensity of 4.5 x 10⁶.

In Figure 3D, an example of the deconvoluted mass spectrum of peaks is shown. It comes from the displayed offline measurement and differs only in intensity and peak form from the results of the other methods.

6 modifications were detected in all methods, including the loss of an N-acetylglucosamine building block and the loss of fucose at N-glycosylation.

In addition, various variations of galactosylation of N-glycosylation have been found, such as GOF/GOF, G0F/G1F, G1F/G1F and G1F/G2F. All found and not found modifications to the different methods are listed in the table below. The table lists all possible glycosylations of a native antibody. Found forms of glycosylation are marked with a tick and unfound with a cross.

| **Modification** | **Offline Probe** | **Online Probe** | **3-in-1 method** |
|---|---|---|---|
| G0F/G0F - GlcNAc | ✔ | ✔ | ✔ |
| G0F/G0F - Fuc | ✔ | ✔ | ✔ |
| G0F/G0F - Light | 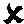 | 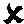 | 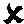 |
| G0F/G0F | ✔ | ✔ | ✔ |
| G0F/G1F | ✔ | ✔ | ✔ |
| G1F/G1F | ✔ | ✔ | ✔ |
| G1F/G2F | ✔ | ✔ | ✔ |
| G2F/G2F | 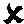 | 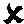 | 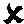 |
| G2F/G2F + NeuAc | 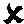 | 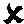 | 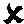 |

It is clear that the method of the invention provides comparable results to the known offline and online measurements.

### Native Measurements of mAb2

For the bi-specific antibody, a size exclusion chromatography was performed to separate the high and low molecular weight fragments of the mAb. A chromatogram of the separation was recorded using variable wave detector and is shown in Figure 4. For the online and 3 in 1 method, only the light molecular weight (LMW), peak 1 to peak 4, were cut. These are located behind the main peak peak of Peak 1. Peak 1 represents the intact antibody, while peak 2 visualizes the Fab/c knob (button), Peak 3 the Fab/c hole and Peak 4 the Fab part of the bispecific antibody.

Figure 5 shows the native measurements of the mAb2. Figure 5A is the offline measurement in which a peak can be seen at minute 9 with an intensity of 2.5 x 10⁶. Figure 5B shows the native online measurement, in which a peak at minute 11 with an intensity of 4 x 10⁶ can be seen. The measurement of the 3 in 1 method is shown in Figure 5C which has a peak at minute 13.

Figure 5D shows the deconvoluted mass spectrum of the mAb2 of the online measurement as an example. On display are the modifications of the N-glycosylation of mAb2, which would be the loss of a fucose molecule and the galactosylation variants GOF/GOF, G0F/G1F, G1F/G1F and G1F/G2F. The same modifications were observed for each of he three methods.

All found and not found modifications of the different methods are listed in the table below. The table lists all possible modifications of a native antibody. Found modifications are marked with a tick and unfound modifications with a cross.

| **Modification** | **Offline Probe** | **Online Probe** | **3-in-1 method** |
|---|---|---|---|
| G0F/G0F - GlcNAc | ✔ | ✔ | ✔ |
| G0F/G0F - Fuc | 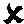 | 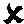 | 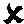 |
| G0F/G0F - Light | 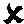 | 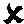 | 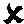 |
| G0F/G0F | ✔ | ✔ | ✔ |
| G0F/G1F | ✔ | ✔ | ✔ |
| G1F/G1F | ✔ | ✔ | ✔ |
| G1F/G2F | ✔ | ✔ | ✔ |
| G2F/G2F | 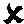 | 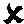 | 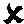 |
| G2F/G2F + NeuAc | 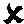 | 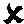 | 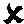 |

It is clear that the method of the invention provides comparable results to the known offline and online measurements.

### Reduced Measurements of mAb 1

Figure 6 shows the chromatograms of the reduced measurements of the mAb1 with all methods, as well as the deconvoluted mass spectra of the light and heavy chain.

In Figure 6A, the offline chromatogram is shown with a peak at minute 5.4 with an intensity of 8 x 10⁷ and a peak at minute 6.8 with a lower intensity of 4 x 10⁷. In the mass spectrum of the first peak, at minute 5.4, the light chain of the mAb1 (Figure 6D) could be identified and at the second peak the heavy chain (Figure 6E).

The online chromatogram is shown in Figure 6B. Here are two peaks at minute 4.5 with an intensity of over 8 x 10⁷ and an upstream secondary peak with an intensity of 6 x 10⁷. In addition, there is a peak at minute 5.3 with an intensity of 6 x 10⁷. In addition, there is a strong baseline increase from 1 x 10⁷ to 4 x 107 from the second peak. In the mass spectrum of the first peak at minute 4.5 the light chain could be identified and in the mass spectrum of the secondary tip also the light chain with a mass shift of -2 Dalton. The heavy chain of the mAb1 could be identified at the peak at minute 5.3.

Figure 6C shows the reduced measurement of the mAb1 of the 3 in 1 method. In the chromatogram, a peak with an intensity of 2.5 x 10 7 was identified at minute 3.8, which was caused by the light chain, and at minute 6.8 also with an intensity of 2.5 x 10⁷. In the mass spectrum of the second peak, the heavy chain was identified. Again, there is a strong baseline increase after the first peak from minute 4.

Figure 6D shows the deconvoluted mass spectrum of the light chain from offline measurement. No modifications were identified. The peak pattern in Figure 6E shows the heavy chain with its modifications. The loss of fucose at N-glycosylation was demonstrated as well as the various galactosylations of N-glycosylations G0F, G1F and G2F. The same modifications were observed for each of the three methods.

All found and not found modifications are listed in the table below. The table shows the Light chain of the mAb1 in the first row and modification of the heavy chain C in the remaining rows. The modifications G0, G0F, G1F, G2F were found. Found modifications are marked with a tick and unfound modifications with a cross.

| **Modification** | **Offline Probe** | **Online Probe** | **3-in-1 Probe** |
|---|---|---|---|
| Light Chain | ✔ | ✔ | ✔ |
| HC Lys | 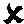 | 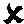 | 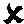 |
| HC G0F-GlcNAc | 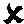 | 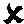 | 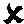 |
| HC G0 | ✔ | ✔ | ✔ |
| HC Man5F | 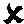 | 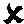 | 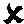 |
| HC G0F | ✔ | ✔ | ✔ |
| HC G0F+Lys | 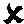 | 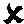 | 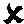 |
| HC G1F | ✔ | ✔ | ✔ |
| HC G2F | ✔ | ✔ | ✔ |

It is clear that the method of the invention provides comparable results to the known offline and online measurements.

### Reduced Measurements of mAb2

Figure 7 shows the chromatograms of the reduced measurements of the mAb2 with all methods, as well as the deconvoluted mass spectra of the light and heavy chain.

In Figure 7A, the chromatogram of the offline measurement of the reduced mAb2 is shown. The first peak at minute 5 has an intensity of 8 x 10⁷, the second peak at minute 6.5 has an intensity of 6 x 10⁷ and the third peak at minute 7 has an intensity of 4 x 10⁷. In the mass spectrum of the first peak the LC A could be identified, in the second peak the LC B and the HC H and in the mass spectrum of the last peak the HC K.

Figure 7B shows the online chromatogram with the first peak at minute 7.5 with an intensity of 3 x 10⁷, the second peak at minute 11 with an intensity of 2 x 10⁷ and a third peak at minute 12 with an intensity of 3 x 10⁷. In the mass spectra of the peaks, the chains of the mAb2 could be identified in the same order. In the mass spectrum of the first peak the LC A, in the mass spectrum of the second peak the LC B and HC H and in the third peak the HC K.

Figure 7C shows the reduced measurement of the mAb2 of the 3 in 1 method. In the chromatogram, the first peak is at minute 7 with an intensity of 3 x 10⁷ and the second peak at minute 13.5 also with an intensity of 3 x 10⁷.

In Figures 7D to 7G, the chains of the mAb2 with their modifications are shown as examples on the basis of the online measurement. In Figure7D and 7E the two different lightweight chains of the mAb2 are shown. The LC A (Figure 7D) was found in the mass spectrum of the first peak and the LC B in Figure 7E in the second peak. In Figure 7F, the heavy chain H is shown with the modifications. It was also discovered in the mass spectrum of the second peak. In HC H, the modification G0F-GlcNAc, i.e. the loss of an N-acetylglucosamine at N-glycosylation, was found, as well as the galactosylation variations G0F and G1F. In the mass spectrum of the third peak, hc K was found with its modifications G0, i.e. the loss of fucose at N-glycosylation, and the galactosylation variants G0F and G1F. The same modifications were observed for each of the three methods.

All found and not found modifications of the different methods are in the table below. The table shows all possible modifications of the reduced antibody. The table shows Light chain A and B of the mAb2 in the first two rows: modifications of the heavy chain H of the mAb2. G0F-GlcNAc, G0F, G1F; and modifications of the heavy chain K. G0, G0F and G1F were found. Found modifications are marked with a tick and unfound modifications with a cross.

| **Modification** | **Offline Probe** | **Online Probe** | **3-in-1 Probe** | |
|---|---|---|---|---|
| Light Chain A | ✔ | ✔ | ✔ | Light chains |
| Light Chain B | ✔ | ✔ | ✔ | |
| HC H Lys | 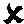 | 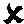 | 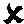 | Heavy chain H modifications |
| HC H G0F-GlcNAc | ✔ | ✔ | ✔ | |
| HC H G0 | 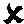 | 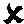 | 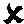 | |
| HC H Man5F | 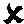 | 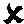 | 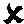 | |
| HC H G0F | ✔ | ✔ | ✔ | |
| HC H G0F+Lys | 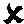 | 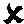 | 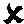 | |
| HC H G1F | ✔ | ✔ | ✔ | |
| HC H G2F | 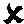 | 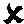 | 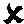 | |
| HC K G0F-GlcNAc | 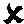 | 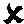 | 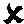 | Modification of the Heavy chain K |
| HC K G0 | ✔ | ✔ | ✔ | |
| HC K Man5F | 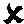 | 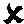 | 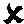 | |
| HC K G0F | ✔ | ✔ | ✔ | |
| HC K GOF+Lys | 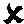 | 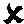 | 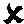 | |
| HC K G1F | ✔ | ✔ | ✔ | |
| HC K G2F | 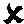 | 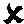 | 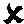 | |

It is clear that the method of the invention provides comparable results to the known offline and online measurements.

### Peptide Mapping of mAb 1

Figure 8 shows the chromatograms of the peptide mappings of the mAb1 of all methods. Figure 8A is the chromatogram of the offline method, Figure 8B is the chromatogram of the online method and Figure 8C is the chromatogram of the 3 in 1 method of the invention.

In Figure 8A, there are many peaks in the anterior part of the chromatogram, i.e. from minute 0 to minute 11. In contrast to the online chromatogram (Figure 8B) and 3 in 1 chromatogram (Figure 8C) where there are only a few peaks in this area. On the other hand, there are significantly more peaks in the range from minute 11 to minute 31 in Figures 8B and 8C compared to 8A.

In the offline method, the average intensity of the peaks is in the range of 0.4 to 0.5 x 10⁸, while the average intensity of the online and 3 in 1 method is 1.5 x 10⁸ or 1.0 x 10⁸. The most intense signal in the offline chromatogram has an intensity of 1 x 10⁸ at minute 24. In the online chromatogram, the most intense signal is at minute 21 with an intensity of 2.6 x 10⁸. In Figure 8C, that of the 3 in 1 method, the most intense signal is at minute 35 with an intensity of 2 x 10⁸.

The table below summarised the highest sequence coverage (SC) of the mAb1 of the three methods.

| **Antibody** | **Offline Probe SC [%]** | **Online Probe SC [%]** | **3-in-1 Probe SC [%]** |
|---|---|---|---|
| mAb1 | 100 | 93.42 | 90.36 |

It is clear that the method of the invention provides comparable results to the known online measurements.

### Peptide Mapping of mAb2

Figure 9 depicts the chromatograms of the peptide mappings of the LMW 4 of the mAb2 in all methods. Thus, only the peptide mapping of the Fab hole part is shown. Figure 9A is the chromatogram of the offline method, Figure 9B is the chromatogram of the online method and Figure 9C is the chromatogram of the 3 in 1 method of the invention.

In Figure 9A, the average intensity is 0.5 x 10⁸ and most signals are in the range of minute 0 to minute 10, while in the back of the chromatogram from minute 25 there are hardly any signals. The most intense signal is at minute 17.5 with an intensity of 0.9 x 10⁸. Figure B and C, there are little or no signals in the front range from minute 0 to minute 10. In the middle range of chromatograms from minute 12 to minute 27 Figures 9B and C have more peaks that Figure 9A. The average signal height is 0.75 x 10⁸ for the online method and 0.4 x 10⁸ for the 3 in 1 method.

The table below summarised the highest sequence coverage (SC) of the mAb2 of the three methods.

| **Antibody** | **Offline Probe SC [%]** | **Online Probe SC [%]** | **3-in-1 Probe SC [%]** |
|---|---|---|---|
| mAb2 | 98.25 | 95.50 | 94.00 |

It is clear that the method of the invention provides comparable results to the known online measurements.

## Claims

1. A multidimensional liquid chromatography (LC) system comprising
a fractionation module having a fractionation column for providing fractionated samples
a first splitter fluidly connectable to and downstream from the fractionation module and fluidly connectable to and upstream from a reduction module wherein the first splitter has an inlet, a first outlet and a second outlet for splitting the fractionated samples into a first measurement sample, such as a native antibody measurement sample, that is passed through the first outlet and a sample for reduction that is passed through the second outlet
the reduction module having a reduction column fluidly connectable to the second outlet of the first splitter
a second splitter fluidly connectable to and downstream from the reduction module and fluidly connectable to and upstream from a digestion module wherein the second splitter has an inlet, a first outlet and a second outlet for splitting the reduced sample into a second measurement sample, such as a reduced antibody measurement sample, that is passed through the first outlet, and a sample for digestion that is passed through the second outlet
the digestion module having a digestion column containing an immobilised proteolytic enzyme for digesting the reduced sample to provide a digested sample
wherein the digestion column is fluidly connectable to the second outlet of the second splitter
a separation module having a separation column for separating analytes and providing a third measurement sample, such as a peptide mapping sample.

2. The multidimensional LC system of claim 1 further comprising a buffer exchange module having a buffer exchange column.

3. The multidimensional LC system of claim 2 wherein the buffer exchange column is a size exclusion chromatography column or a reverse phase chromatography column.

4. The multidimensional LC system of any one claim 2 or 3 wherein the buffer exchange module is downstream from the fractionation module and upstream from and fluidly connectable to the first splitter.

5. The multidimensional LC system of any one of the preceding claims wherein the system has a first valve assembly configured so that in a first position:
the inlet of the first splitter is fluidly connected to the fractionation module and, when present, the buffer exchange module or a multiple heart cutting valve;
the first outlet of the first splitter is fluidly connected to another module of the system such that a first measurement sample can be split from the flow;
and the second outlet of the first splitter is fluidly connected to the reduction module,
and in a second position the inlet and the second outlet of the first splitter are connected in a loop and the reduction module is fluidly connected to the second splitter and when present a reduction pump.

6. The multidimensional LC system of any one of the preceding claims wherein the system has a second valve assembly configured so that in a first position the second outlet of the second splitter is fluidly connected to the digestion module and, when present, a trapping module and in a second position of the second valve assembly, the digestion module is isolated and is not in fluid connected with any other components

7. The multidimensional LC system of any one of the preceding claims wherein the system has a fourth valve assembly configured so that in a first position the first outlet of the first splitter is fluidly connected to another module of the system and so that in a second position the first outlet of the second splitter is fluidly connectable to another module of the system optionally wherein the another module may be an analysis module such as a mass spectrometer.

8. The multidimensional LC system of any one of the preceding claims wherein the system has a third valve assembly configured so that the first outlet of the second splitter is connected to another module of the system when the third valve assembly is in the first position and the separation module is fluidly connected to another module of the system when the third valve assembly is in the second position, preferably in both cases the fourth valve assembly is in the second position.

9. The multidimensional LC system of any one of the preceding claims further comprising a multiple heart cutting valve fluidly connected to the fractionation column and is after the fractionation column in the direction of flow.

10. The multidimensional LC system of any one of the previous claims wherein the digestion module has a fourth splitter such as static mixer or a zero delay volume T-Piece downstream from digestion column in the direction of flow or a third splitter such as static mixer or a zero delay volume T-Piece upstream from the digestion column in the direction of flow.

11. The multidimensional LC system of any one of the previous claims further comprising an analysis module for analysing the first measurement sample, the second measurement sample and the third measurement sample optionally wherein the analysis module comprises a mass spectrometer (HRMS) or a Single Quad mass spectrometer; an evaporative light scattering detector (ELSD): a UV detector; or a diode array detector (DAD).

12. A multidimensional LC process for analysing a sample of therapeutic antibodies comprising the steps of:
providing a sample of antibodies to be analysed:
introducing the sample to the system of any one of claims 1 to 11.

13. The multidimensional LC process of claim 12 wherein
the sample is flowed through the fractionation module and the first splitter;
at the first splitter, the sample is split into a first and second flow;
the first flow is the first measurement sample and is flowed to an analysis module;
the second flow is flowed to the reduction module and is reduced before passing to the second splitter;
at the second splitter, the sample is split into a third and fourth flow;
the third flow is the second measurement sample and is flowed to the analysis module; and
the fourth flow is flowed to the digested module and is digested before being separated in the separation module to provide the third measurements sample.

14. The multidimensional LC process of claim 13 wherein a first, second, third and fourth valve assembly are used to control when each of the first, second and third measurement samples are sent for analysis for example to an analysis module.

15. The multidimensional LC process of claim 14 wherein the process comprises the following steps in order:
a. The sample is introduced to the system and fractionated by the fractionation module, the fractions are optionally stored in a multiple heart cutting valve.
b. Optionally, the fractions are flowed through a digestion module to the first splitter.
c. The first valve assembly is in the first position, the second valve assembly is in the first position and the fourth valve assembly is in the first position so the first measurement sample is separated and analyzed and the rest of the sample is loaded onto the reduction column.
d. The first valve assembly is switched to the second position and the sample is reduced.
e. After the sample is reduced, the second valve is switched to the second position and the fourth valve assembly is switched to the second position and the third valve assembly is in the first position.
f. The reduced sample is flushed off the reduction column to the second splitter so the second measurement sample can be separated and passed through the third and fourth valve assemblies to be analyzed and the rest of the reduced sample is passed through the digestion column to a trapping column if present or directly to the separation column if no trapping column is present.
g. If the trapping column is present, the third valve assembly is in the first position when the digested sample is trapped and is then switched to the second position so that the digested sample can be passed to the separation column.

## Patentansprüche

1. Mehrdimensionales Flüssigkeitschromatographie (LC)-System, umfassend
ein Fraktionierungsmodul mit einer Fraktionierungssäule zum Bereitstellen fraktionierter Proben,
einen ersten Verteiler, der fluidisch mit dem Fraktionierungsmodul verbunden werden kann und diesem nachgelagert ist und fluidisch mit einem Reduktionsmodul verbunden werden kann und diesem vorgelagert ist, wobei der erste Verteiler einen Einlass, einen ersten Auslass und einen zweiten Auslass zum Aufteilen der fraktionierten Proben in eine erste Messprobe, wie beispielsweise eine Messprobe eines nativen Antikörpers, die durch den ersten Auslass geleitet wird, und eine Probe zur Reduktion, die durch den zweiten Auslass geleitet wird, aufweist,
wobei das Reduktionsmodul eine Reduktionssäule aufweist, die fluidisch mit dem zweiten Auslass des ersten Verteilers verbunden werden kann,
einen zweiten Verteiler, der fluidisch mit dem Reduktionsmodul verbunden werden kann und diesem nachgelagert ist und fluidisch mit einem Verdaumodul verbunden werden kann und diesem vorgelagert ist, wobei der zweite Verteiler einen Einlass, einen ersten Auslass und einen zweiten Auslass zum Aufteilen der reduzierten Probe in eine zweite Messprobe, wie beispielsweise eine Messprobe eines reduzierten Antikörpers, die durch den ersten Auslass geleitet wird, und eine Probe zum Verdau, die durch den zweiten Auslass geleitet wird, aufweist,
wobei das Verdaumodul eine Verdausäule aufweist, die ein immobilisiertes proteolytisches Enzym zum Verdauen der reduzierten Probe enthält, um eine verdaute Probe bereitzustellen,
wobei die Verdausäule fluidisch mit dem zweiten Auslass des zweiten Verteilers verbunden werden kann,
ein Trennungsmodul mit einer Trennungssäule zum Trennen von Analyten und Bereitstellen einer dritten Messprobe, wie beispielsweise einer Peptid-Mapping-Probe.

2. Mehrdimensionales LC-System nach Anspruch 1, ferner umfassend ein Pufferaustauschmodul mit einer Pufferaustauschsäule.

3. Mehrdimensionales LC-System nach Anspruch 2, wobei die Pufferaustauschsäule eine Größenausschlusschromatographiesäule oder eine Umkehrphasenchromatographiesäule ist.

4. Mehrdimensionales LC-System nach einem der Ansprüche 2 oder 3, wobei das Pufferaustauschmodul dem Fraktionierungsmodul nachgelagert und dem ersten Verteiler vorgelagert ist und fluidisch mit diesem verbunden werden kann.

5. Mehrdimensionales LC-System nach einem der vorhergehenden Ansprüche, wobei das System eine erste Ventilanordnung aufweist, die so konfiguriert ist, dass in einer ersten Position:
der Einlass des ersten Verteilers fluidisch mit dem Fraktionierungsmodul und, wenn vorhanden, dem Pufferaustauschmodul oder einem Multiple-Heart-Cutting-Ventil verbunden ist;
der erste Auslass des ersten Verteilers fluidisch mit einem anderen Modul des Systems verbunden ist, sodass eine erste Messprobe aus dem Fluss abgeteilt werden kann;
und der zweite Auslass des ersten Verteilers fluidisch mit dem Reduktionsmodul verbunden ist,
und in einer zweiten Position der Einlass und der zweite Auslass des ersten Verteilers in einer Schleife verbunden sind und das Reduktionsmodul fluidisch mit dem zweiten Verteiler und, wenn vorhanden, einer Reduktionspumpe verbunden ist.

6. Mehrdimensionales LC-System nach einem der vorhergehenden Ansprüche, wobei das System eine zweite Ventilanordnung aufweist, die so konfiguriert ist, dass in einer ersten Position der zweite Auslass des zweiten Verteilers fluidisch mit dem Verdaumodul und, wenn vorhanden, einem Auffangmodul verbunden ist und in einer zweiten Position der zweiten Ventilanordnung das Verdaumodul isoliert ist und nicht fluidisch mit anderen Komponenten verbunden ist.

7. Mehrdimensionales LC-System nach einem der vorhergehenden Ansprüche, wobei das System eine vierte Ventilanordnung aufweist, die so konfiguriert ist, dass in einer ersten Position der erste Auslass des ersten Verteilers fluidisch mit einem anderen Modul des Systems verbunden ist und in einer zweiten Position der erste Auslass des zweiten Verteilers fluidisch mit einem anderen Modul des Systems verbunden werden kann, wobei das andere Modul optional ein Analysemodul wie z. B. ein Massenspektrometer sein kann.

8. Mehrdimensionales LC-System nach einem der vorhergehenden Ansprüche, wobei das System eine dritte Ventilanordnung aufweist, die so konfiguriert ist, dass der erste Auslass des zweiten Verteilers mit einem anderen Modul des Systems verbunden ist, wenn sich die dritte Ventilanordnung in der ersten Position befindet, und das Trennungsmodul fluidisch mit einem anderen Modul des Systems verbunden ist, wenn sich die dritte Ventilanordnung in der zweiten Position befindet, wobei sich die vierte Ventilanordnung vorzugsweise in beiden Fällen in der zweiten Position befindet.

9. Mehrdimensionales LC-System nach einem der vorhergehenden Ansprüche, ferner umfassend ein Multiple-Heart-Cutting-Ventil, das fluidisch mit der Fraktionierungssäule verbunden ist und sich in Strömungsrichtung hinter der Fraktionierungssäule befindet.

10. Mehrdimensionales LC-System nach einem der vorhergehenden Ansprüche, wobei das Verdaumodul einen vierten Verteiler, wie z. B. einen statischen Mischer oder ein Null-Verzögerungsvolumen-T-Stück, der der Verdausäule in Strömungsrichtung nachgelagert ist, oder einen dritten Verteiler, wie z. B. einen statischen Mischer oder ein Null-Verzögerungsvolumen-T-Stück, der der Verdausäule in Strömungsrichtung vorgelagert ist, aufweist.

11. Mehrdimensionales LC-System nach einem der vorhergehenden Ansprüche, ferner umfassend ein Analysemodul zum Analysieren der ersten Messprobe, der zweiten Messprobe und der dritten Messprobe, wobei das Analysemodul optional ein Massenspektrometer (HRMS) oder ein Single-Quad-Massenspektrometer; einen Verdampfungslichtstreuungsdetektor (ELSD); einen UV-Detektor; oder einen Diodenarraydetektor (DAD) umfasst.

12. Mehrdimensionales LC-Verfahren zum Analysieren einer Probe von therapeutischen Antikörpern, umfassend die folgenden Schritte:
Bereitstellen einer Probe von zu analysierenden Antikörpern;
Einführen der Probe in das System nach einem der Ansprüche 1 bis 11.

13. Mehrdimensionales LC-Verfahren nach Anspruch 12, wobei
die Probe durch das Fraktionierungsmodul und den ersten Verteiler geleitet wird;
die Probe am ersten Verteiler in einen ersten und einen zweiten Fluss aufgeteilt wird;
der erste Fluss die erste Messprobe ist und zu einem Analysemodul geleitet wird;
der zweite Fluss zum Reduktionsmodul geleitet wird und reduziert wird, bevor er zum zweiten Verteiler geleitet wird;
die Probe am zweiten Verteiler in einen dritten und einen vierten Fluss aufgeteilt wird;
der dritte Fluss die zweite Messprobe ist und zum Analysemodul geleitet wird; und
der vierte Fluss zum verdauten Modul geleitet wird und verdaut wird, bevor er im Trennungsmodul getrennt wird, um die dritte Messprobe bereitzustellen.

14. Mehrdimensionales LC-Verfahren nach Anspruch 13, wobei eine erste, zweite, dritte und vierte Ventilanordnung verwendet werden, um zu steuern, wann jede der ersten, zweiten und dritten Messproben zur Analyse, beispielsweise an ein Analysemodul, gesendet wird.

15. Mehrdimensionales LC-Verfahren nach Anspruch 14, wobei das Verfahren die folgenden Schritte in der Reihenfolge umfasst:
a. Die Probe wird in das System eingeführt und durch das Fraktionierungsmodul fraktioniert, wobei die Fraktionen optional in einem Multiple-Heart-Cutting-Ventil gespeichert werden.
b. Die Fraktionen werden optional durch ein Verdaumodul zum ersten Verteiler geleitet.
c. Die erste Ventilanordnung befindet sich in der ersten Position, die zweite Ventilanordnung befindet sich in der ersten Position und die vierte Ventilanordnung befindet sich in der ersten Position, sodass die erste Messprobe getrennt und analysiert wird und der Rest der Probe auf die Reduktionssäule geladen wird.
d. Die erste Ventilanordnung wird in die zweite Position geschaltet und die Probe wird reduziert.
e. Nachdem die Probe reduziert wurde, wird das zweite Ventil in die zweite Position geschaltet und die vierte Ventilanordnung wird in die zweite Position geschaltet und die dritte Ventilanordnung befindet sich in der ersten Position.
f. Die reduzierte Probe wird von der Reduktionssäule zum zweiten Verteiler gespült, sodass die zweite Messprobe getrennt und durch die dritte und vierte Ventilanordnung geleitet werden kann, um analysiert zu werden, und der Rest der reduzierten Probe wird durch die Verdausäule zu einer Auffangsäule geleitet, falls vorhanden, oder direkt zur Trennungssäule, falls keine Auffangsäule vorhanden ist.
g. Falls die Auffangsäule vorhanden ist, befindet sich die dritte Ventilanordnung in der ersten Position, wenn die verdaute Probe aufgefangen wird, und wird dann in die zweite Position geschaltet, sodass die verdaute Probe zur Trennungssäule geleitet werden kann.

## Revendications

1. Système de chromatographie liquide (LC) multidimensionnel comprenant
un module de fractionnement ayant une colonne de fractionnement pour fournir des échantillons fractionnés
un premier diviseur pouvant être raccordé de manière fluidique au et en aval du module de fractionnement et pouvant être raccordé de manière fluidique à et en amont d'un module de réduction dans lequel le premier diviseur a une entrée, une première sortie et une seconde sortie pour diviser les échantillons fractionnés en un premier échantillon de mesure, tel qu'un échantillon de mesure d'anticorps natifs, qui passe à travers la première sortie et un échantillon pour réduction qui passe à travers la seconde sortie
le module de réduction ayant une colonne de réduction pouvant être raccordée de manière fluidique à la seconde sortie du premier diviseur
un deuxième diviseur pouvant être raccordé de manière fluidique au et en aval du module de réduction et pouvant être raccordé de manière fluidique à et en amont d'un module de digestion dans lequel le deuxième diviseur a une entrée, une première sortie et une seconde sortie pour diviser l'échantillon réduit en un deuxième échantillon de mesure, tel qu'un échantillon de mesure d'anticorps réduits, qui passe à travers la première sortie, et un échantillon pour digestion qui passe à travers la seconde sortie
le module de digestion ayant une colonne de digestion contenant une enzyme protéolytique immobilisée pour digérer l'échantillon réduit afin de fournir un échantillon digéré
dans lequel la colonne de digestion peut être raccordée de manière fluidique à la seconde sortie du deuxième diviseur
un module de séparation ayant une colonne de séparation pour séparer les analytes et fournir un troisième échantillon de mesure, tel qu'un échantillon de cartographie peptidique.

2. Système LC multidimensionnel selon la revendication 1 comprenant en outre un module d'échange de tampon ayant une colonne d'échange de tampon.

3. Système LC multidimensionnel selon la revendication 2 dans lequel la colonne d'échange de tampon est une colonne de chromatographie d'exclusion stérique ou une colonne de chromatographie en phase inverse.

4. Système LC multidimensionnel selon une quelconque revendication 2 ou 3 dans lequel le module d'échange de tampon est en aval du module de fractionnement et en amont du et pouvant être raccordé de manière fluidique au premier diviseur.

5. Système LC multidimensionnel selon l'une quelconque des revendications précédentes dans lequel le système a un premier ensemble vanne conçu de sorte que dans une première position :
l'entrée du premier diviseur est raccordée de manière fluidique au module de fractionnement et, lorsqu'il est présent, au module d'échange de tampon ou à une vanne de sélection multi-voies ;
la première sortie du premier diviseur est raccordée de manière fluidique à un autre module du système de telle sorte qu'un premier échantillon de mesure peut être divisé à partir de l'écoulement ;
et la seconde sortie du premier diviseur est raccordée de manière fluidique au module de réduction,
et dans une seconde position, l'entrée et la seconde sortie du premier diviseur sont raccordées en une boucle et le module de réduction est raccordé de manière fluidique au deuxième diviseur et lorsqu'elle est présente à une pompe de réduction.

6. Système LC multidimensionnel selon l'une quelconque des revendications précédentes dans lequel le système a un deuxième ensemble vanne conçu de sorte que dans une première position, la seconde sortie du deuxième diviseur est raccordée de manière fluidique au module de digestion et, lorsqu'il est présent, à un module de piégeage et dans une seconde position du deuxième ensemble vanne, le module de digestion est isolé et n'est pas raccordé de manière fluidique à un quelconque autre composant.

7. Système LC multidimensionnel selon l'une quelconque des revendications précédentes dans lequel le système a un quatrième ensemble vanne conçu de sorte que dans une première position, la première sortie du premier diviseur est raccordée de manière fluidique à un autre module du système et de sorte que dans une seconde position, la première sortie du deuxième diviseur peut être raccordée de manière fluidique à un autre module du système éventuellement dans lequel l'autre module peut être un module d'analyse tel qu'un spectromètre de masse.

8. Système LC multidimensionnel selon l'une quelconque des revendications précédentes dans lequel le système a un troisième ensemble vanne conçu de sorte que la première sortie du deuxième diviseur est raccordée à un autre module du système lorsque le troisième ensemble vanne est dans la première position et le module de séparation est raccordé de manière fluidique à un autre module du système lorsque le troisième ensemble vanne est dans la seconde position, de préférence dans les deux cas le quatrième ensemble vanne est dans la seconde position.

9. Système LC multidimensionnel selon l'une quelconque des revendications précédentes comprenant en outre une vanne de sélection multi-voies raccordée de manière fluidique à la colonne de fractionnement et est après la colonne de fractionnement dans le sens de l'écoulement.

10. Système LC multidimensionnel selon l'une quelconque des revendications précédentes dans lequel le module de digestion a un quatrième diviseur tel qu'un mélangeur statique ou une pièce en T sans volume de retard en aval de la colonne de digestion dans le sens de l'écoulement ou un troisième diviseur tel qu'un mélangeur statique ou une pièce en T sans volume de retard en amont de la colonne de digestion dans le sens de l'écoulement.

11. Système LC multidimensionnel selon l'une quelconque des revendications précédentes comprenant en outre un module d'analyse pour analyser le premier échantillon de mesure, le deuxième échantillon de mesure et le troisième échantillon de mesure éventuellement dans lequel le module d'analyse comprend un spectromètre de masse (HRMS) ou un spectromètre de masse à simple quadripôle ; un détecteur évaporatif à diffusion de lumière (ELSD) ; un détecteur UV ; ou un détecteur à barrette de diodes (DAD).

12. Procédé LC multidimensionnel pour analyser un échantillon d'anticorps thérapeutiques comprenant les étapes de :
fourniture d'un échantillon d'anticorps à analyser :
introduction de l'échantillon dans le système selon l'une quelconque des revendications 1 à 11.

13. Procédé LC multidimensionnel selon la revendication 12 dans lequel
l'échantillon s'écoule à travers le module de fractionnement et le premier diviseur ;
au niveau du premier diviseur, l'échantillon est divisé en un premier et un deuxième écoulement ;
le premier écoulement est le premier échantillon de mesure et s'écoule jusqu'à un module d'analyse ;
le deuxième écoulement s'écoule jusqu'au module de réduction et est réduit avant de passer dans le deuxième diviseur ;
au niveau du deuxième diviseur, l'échantillon est divisé en un troisième et un quatrième écoulement ;
le troisième écoulement est le deuxième échantillon de mesure et s'écoule jusqu'au module d'analyse ; et
le quatrième écoulement s'écoule jusqu'au module digéré et est digéré avant d'être séparé dans le module de séparation pour fournir le troisième échantillon de mesure.

14. Procédé LC multidimensionnel selon la revendication 13 dans lequel un premier, un deuxième, un troisième et un quatrième ensemble vanne sont utilisés pour commander le moment où chacun des premier, deuxième et troisième échantillons de mesure est envoyé pour analyse par exemple vers un module d'analyse.

15. Procédé LC multidimensionnel selon la revendication 14 dans lequel le procédé comprend les étapes suivantes dans l'ordre :
a. l'échantillon est introduit dans le système et fractionné par le module de fractionnement, les fractions sont éventuellement stockées dans une vanne de sélection multi-voies
b. éventuellement, les fractions s'écoulent à travers un module de digestion jusqu'au premier diviseur
c. le premier ensemble vanne est dans la première position, le deuxième ensemble vanne est dans la première position et le quatrième ensemble vanne est dans la première position de sorte que le premier échantillon de mesure est séparé et analysé et le reste de l'échantillon est chargé sur la colonne de réduction
d. le premier ensemble vanne est commuté dans la seconde position et l'échantillon est réduit
e. après la réduction de l'échantillon, la deuxième vanne est commutée dans la seconde position et le quatrième ensemble vanne est commuté dans la seconde position et le troisième ensemble vanne est dans la première position
f. l'échantillon réduit est purgé de la colonne de réduction jusqu'au deuxième diviseur de sorte que le deuxième échantillon de mesure peut être séparé et passer à travers les troisième et quatrième ensembles vannes pour être analysé et le reste de l'échantillon réduit passe à travers la colonne de digestion jusqu'à une colonne de piégeage si elle est présente ou directement dans la colonne de séparation s'il n'y a pas de colonne de piégeage présente
g. si la colonne de piégeage est présente, le troisième ensemble vanne est dans la première position lorsque l'échantillon digéré est piégé et est ensuite commuté dans la seconde position de sorte que l'échantillon digéré peut passer dans la colonne de séparation.
